# EUROPÄISCHE PATENTSCHRIFT

(11) **EP 1 597 373 B1**
(45) Veröffentlichungstag und Bekanntmachung des Hinweises auf die Patenterteilung: **18.07.2012**
(21) Anmeldenummer: 04711594.4
(22) Anmeldetag: 17.02.2004
(51) Int. Cl.: C12N 15/82, A01H 5/00, C12Q 1/68

(54) **Glyphosat-tolerante Zuckerrübe**
Glyphosate-tolerant sugar beet
Betteraves sucrières tolérant le glyphosate

(30) Priorität: 20.02.2003 EP 03003866; 28.02.2003 US 376763
(43) Veröffentlichungstag der Anmeldung: 23.11.2005
(73) Patentinhaber: KWS Saat AG, 37555 Einbeck (DE)
(72) Erfinder: KRAUS, Josef, D-37574 Einbeck (DE); SAUERBREY, Elke, D-37574 Einbeck (DE); NEHLS, Reinhard, D-37574 Einbeck (DE); LOOCK, Andreas, D-37574 Einbeck (DE); JANSEN, Rudolf, D-37574 Einbeck (DE)
(74) Vertreter: Pohl, Manfred
(86) Internationale Anmeldenummer: PCT/EP2004/001469
(87) Internationale Veröffentlichungsnummer: WO 2004/074492

(56) Entgegenhaltungen:
- WO-A-99/23232

### SEQUENCE LISTING

<110> KWS SAAT AG
<120> Glyphosat-tolerante Zuckerrübe
<130> PCT 0079
<150> EP 03003866.5
   <151> 2003-02-20
<150> US 10/376763
   <151> 2003-02-28
<160> 21
<170> PatentIn version 3.1
<210> 1
   <211> 30
   <212> DNA
   <213> Artificial Sequence
<220>
   <223> Primer
<400> 1
   ttaatttttg caggcgatgg tggctgttat 30
<210> 2
   <211> 30
   <212> DNA
   <213> Artificial Sequence
<220>
   <223> Primer
<400> 2
   catacgcatt agtgagtggg ctgtcaggac 30
<210> 3
   <211> 20
   <212> DNA
   <213> Artificial Sequence
<220>
   <223> Primer
<400> 3
   atgttatctt taccacagtt 20
<210> 4
   <211> 24
   <212> DNA
   <213> Artificial Sequence
<220>
   <223> Primer
<400> 4
   gtccctaaat gaaatacgta aaac 24
<210> 5
   <211> 3778
   <212> DNA
   <213> Artificial Sequence
<220>
   <223> Inserted DNA with 3' and 5' flanking sequences
<400> 5
   ctcgagcggc cgccagtgtg atggatatct gcagaattcg cccttatgtt atctttacca     60
   cagtttgttg ctctgacaca accggtaaat gcattggcct ttgtttttga tggcatcaac    120
   tttggagcat ctgattttgc atattcagcc ttttccatgg taattctttt acaagaattt    180
   tcattctttc ttaagtataa acacttagct tgggacaaac ttctgatcct atttcttaat    240
   ttttgcaggt gatggtggct gttatgagca ttttgtgttt gatgtttctt tcttctcatt 300
   acggttttat tgggatctgg gtggctctaa ctatttacat gagcctccgc gcgtttgctg 360
   aaggcgggaa acgacaatct gatccccatc aagcttgagc tcaggattta gcagcattcc 420
   agattgggtt caatcaacaa ggtacgagcc atatcacttt attcaaattg gtatcgccaa 480
   aaccaagaag gaactcccat cctcaaaggt ttgtaaggaa gaattctcag tccaaagcct 540
   caacaaggtc agggtacaga gtctccaaac cattagccaa aagctacagg agatcaatga 600
   agaatcttca atcaaagtaa actactgttc cagcacatgc atcatggtca gtaagtttca 660
   gaaaaagaca tccaccgaag acttaaagtt agtgggcatc tttgaaagta atcttgtcaa 720
   catcgagcag ctggcttgtg gggaccagac aaaaaaggaa tggtgcagaa ttgttaggcg 780
   cacctaccaa aagcatcttt gcctttattg caaagataaa gcagattcct ctagtacaag 840
   tggggaacaa aataacgtgg aaaagagctg tcctgacagc ccactcacta atgcgtatga 900
   cgaacgcagt gacgaccaca aaagaattcc ctctatataa gaaggcattc attcccattt 960
   gaaggatcat cagatactca accaatcctt ctagaagatc taagcttatc gataagcttg 1020
   atgtaattgg aggaagatca aaattttcaa tccccattct tcgattgctt caattgaagt 1080
   ttctccgatg gcgcaagtta gcagaatctg caatggtgtg cagaacccat ctcttatctc 1140
   caatctctcg aaatccagtc aacgcaaatc tcccttatcg gtttctctga agacgcagca 1200
   gcatccacga gcttatccga tttcgtcgtc gtggggattg aagaagagtg ggatgacgtt 1260
   aattggctct gagcttcgtc ctcttaaggt catgtcttct gtttccacgg cgtgcatgct 1320
   tcacggtgca agcagccgtc cagcaactgc tcgtaagtcc tctggtcttt ctggaaccgt 1380
   ccgtattcca ggtgacaagt ctatctccca caggtccttc atgtttggag gtctcgctag 1440
   cggtgaaacc cgtatcaccg gtcttttgga aggtgaagat gttatcaaca ctggtaaggc 1500
   tatgcaagct atgggtgcca gaatccgtaa ggaaggtgat acttggatca ttgatggtgt 1560
   tggtaacggt ggactccttg ctcctgaggc tcctctcgat ttcggtaacg ctgcaactgg 1620
   ttgccgtttg actatgggtc ttgttggtgt ttacgatttc gatagcactt tcattggtga 1680
   cgcttctctc actaagcgtc caatgggtcg tgtgttgaac ccacttcgcg aaatgggtgt 1740
   gcaggtgaag tctgaagacg gtgatcgtct tccagttacc ttgcgtggac caaagactcc 1800
   aacgccaatc acctacaggg tacctatggc ttccgctcaa gtgaagtccg ctgttctgct 1860
   tgctggtctc aacaccccag gtatcaccac tgttatcgag ccaatcatga ctcgtgacca 1920
   cactgaaaag atgcttcaag gttttggtgc taaccttacc gttgagactg atgctgacgg 1980
   tgtgcgtacc atccgtcttg aaggtcgtgg taagctcacc ggtcaagtga ttgatgttcc   2040
   aggtgatcca tcctctactg ctttcccatt ggttgctgcc ttgcttgttc caggttccga   2100
   cgtcaccatc cttaacgttt tgatgaaccc aacccgtact ggtctcatct tgactctgca   2160
   ggaaatgggt gccgacatcg aagtgatcaa cccacgtctt gctggtggag aagacgtggc   2220
   tgacttgcgt gttcgttctt ctactttgaa gggtgttact gttccagaag accgtgctcc   2280
   ttctatgatc gacgagtatc caattctcgc tgttgcagct gcattcgctg aaggtgctac   2340
   cgttatgaac ggtttggaag aactccgtgt taaggaaagc gaccgtcttt ctgctgtcgc   2400
   aaacggtctc aagctcaacg gtgttgattg cgatgaaggt gagacttctc tcgtcgtgcg   2460
   tggtcgtcct gacggtaagg gtctcggtaa cgcttctgga gcagctgtcg ctacccacct   2520
   cgatcaccgt atcgctatga gcttcctcgt tatgggtctc gtttctgaaa accctgttac   2580
   tgttgatgat gctactatga tcgctactag cttcccagag ttcatggatt tgatggctgg   2640
   tcttggagct aagatcgaac tctccgacac taaggctgct tgatgagctc aagaattcga   2700
   gctcggtacc ggatcctcta gctagagctt tcgttcgtat catcggtttc gacaacgttc   2760
   gtcaagttca atgcatcagt ttcattgcgc acacaccaga atcctactga gtttgagtat   2820
   tatggcattg ggaaaactgt ttttcttgta ccatttgttg tgcttgtaat ttactgtgtt   2880
   ttttattcgg ttttcgctat cgaactgtga aatggaaatg gatggagaag agttaatgaa   2940
   tgatatggtc cttttgttca ttctcaaatt aatattattt gttttttctc ttatttgttg   3000
   tgtgttgaat ttgaaattat aagagatatg caaacatttt gttttgagta aaaatgtgtc   3060
   aaatcgtggc ctctaatgac cgaagttaat atgaggagta aaacacttgt agttgtacca   3120
   ttatgcttat tcactaggca acaaatatat tttcagacct agaaaagctg caaatgttac   3180
   tgaatacaag tatgtcctct tgtgttttag acatttatga actttccttt atgtaatttt   3240
   ccagaatcct tgtcagattc taatcattgc tttataatta tagttatact catggatttg   3300
   tagttgagta tgaaaatatt ttttaatgca ttttatgact tgccaattga ttgacaacat   3360
   gcatcaatcg acctgcagcc actcgaagcg gccgccactc gagtggtggc cgcatcgatc   3420
   gtgaagtttc tcatctaagc ccccatttgg acgtgaatgt agacacgtcg aaataaagat   3480
   ttccgaatta gaataatttg tttattgctt tcgcctataa atacgacgga tcgtaatttg   3540
   tcgttttatc aaaatgtact ttcattttat aataacgctg cggacatcta catttttgaa   3600
   ttgaaaaaaa ttggtaatta ctctttcttt ttctccatat tgaccatcat actcattgct   3660
   gatccatgta gatttcccgg acatgaagcc atttacaatt gaatatatcc taagtaaaac   3720
   ctcataggtt ttacgtattt catttaggga caagggcgaa ttccagcaca ctggcggc   3778
<210> 6
   <211> 3706
   <212> DNA
   <213> Artificial Sequence
<220>
   <223> PCR product
<400> 6
   atgttatctt taccacagtt tgttgctctg acacaaccgg taaatgcatt ggcctttgtt 60
   tttgatggca tcaactttgg agcatctgat tttgcatatt cagccttttc catggtaatt 120
   cttttacaag aattttcatt ctttcttaag tataaacact tagcttggga caaacttctg 180
   atcctatttc ttaatttttg caggtgatgg tggctgttat gagcattttg tgtttgatgt 240
   ttctttcttc tcattacggt tttattggga tctgggtggc tctaactatt tacatgagcc 300
   tccgcgcgtt tgctgaaggc gggaaacgac aatctgatcc ccatcaagct tgagctcagg 360
   atttagcagc attccagatt gggttcaatc aacaaggtac gagccatatc actttattca 420
   aattggtatc gccaaaacca agaaggaact cccatcctca aaggtttgta aggaagaatt 480
   ctcagtccaa agcctcaaca aggtcagggt acagagtctc caaaccatta gccaaaagct 540
   acaggagatc aatgaagaat cttcaatcaa agtaaactac tgttccagca catgcatcat 600
   ggtcagtaag tttcagaaaa agacatccac cgaagactta aagttagtgg gcatctttga 660
   aagtaatctt gtcaacatcg agcagctggc ttgtggggac cagacaaaaa aggaatggtg 720
   cagaattgtt aggcgcacct accaaaagca tctttgcctt tattgcaaag ataaagcaga 780
   ttcctctagt acaagtgggg aacaaaataa cgtggaaaag agctgtcctg acagcccact 840
   cactaatgcg tatgacgaac gcagtgacga ccacaaaaga attccctcta tataagaagg 900
   cattcattcc catttgaagg atcatcagat actcaaccaa tccttctaga agatctaagc 960
   ttatcgataa gcttgatgta attggaggaa gatcaaaatt ttcaatcccc attcttcgat 1020
   tgcttcaatt gaagtttctc cgatggcgca agttagcaga atctgcaatg gtgtgcagaa 1080
   cccatctctt atctccaatc tctcgaaatc cagtcaacgc aaatctccct tatcggtttc 1140
   tctgaagacg cagcagcatc cacgagctta tccgatttcg tcgtcgtggg gattgaagaa 1200
   gagtgggatg acgttaattg gctctgagct tcgtcctctt aaggtcatgt cttctgtttc 1260
   cacggcgtgc atgcttcacg gtgcaagcag ccgtccagca actgctcgta agtcctctgg 1320
   tctttctgga accgtccgta ttccaggtga caagtctatc tcccacaggt ccttcatgtt 1380
   tggaggtctc gctagcggtg aaacccgtat caccggtctt ttggaaggtg aagatgttat 1440
   caacactggt aaggctatgc aagctatggg tgccagaatc cgtaaggaag gtgatacttg 1500
   gatcattgat ggtgttggta acggtggact ccttgctcct gaggctcctc tcgatttcgg 1560
   taacgctgca actggttgcc gtttgactat gggtcttgtt ggtgtttacg atttcgatag 1620
   cactttcatt ggtgacgctt ctctcactaa gcgtccaatg ggtcgtgtgt tgaacccact 1680
   tcgcgaaatg ggtgtgcagg tgaagtctga agacggtgat cgtcttccag ttaccttgcg 1740
   tggaccaaag actccaacgc caatcaccta cagggtacct atggcttccg ctcaagtgaa 1800
   gtccgctgtt ctgcttgctg gtctcaacac cccaggtatc accactgtta tcgagccaat 1860
   catgactcgt gaccacactg aaaagatgct tcaaggtttt ggtgctaacc ttaccgttga 1920
   gactgatgct gacggtgtgc gtaccatccg tcttgaaggt cgtggtaagc tcaccggtca 1980
   agtgattgat gttccaggtg atccatcctc tactgctttc ccattggttg ctgccttgct 2040
   tgttccaggt tccgacgtca ccatccttaa cgttttgatg aacccaaccc gtactggtct 2100
   catcttgact ctgcaggaaa tgggtgccga catcgaagtg atcaacccac gtcttgctgg 2160
   tggagaagac gtggctgact tgcgtgttcg ttcttctact ttgaagggtg ttactgttcc 2220
   agaagaccgt gctccttcta tgatcgacga gtatccaatt ctcgctgttg cagctgcatt 2280
   cgctgaaggt gctaccgtta tgaacggttt ggaagaactc cgtgttaagg aaagcgaccg 2340
   tctttctgct gtcgcaaacg gtctcaagct caacggtgtt gattgcgatg aaggtgagac 2400
   ttctctcgtc gtgcgtggtc gtcctgacgg taagggtctc ggtaacgctt ctggagcagc 2460
   tgtcgctacc cacctcgatc accgtatcgc tatgagcttc ctcgttatgg gtctcgtttc 2520
   tgaaaaccct gttactgttg atgatgctac tatgatcgct actagcttcc cagagttcat 2580
   ggatttgatg gctggtcttg gagctaagat cgaactctcc gacactaagg ctgcttgatg 2640
   agctcaagaa ttcgagctcg gtaccggatc ctctagctag agctttcgtt cgtatcatcg 2700
   gtttcgacaa cgttcgtcaa gttcaatgca tcagtttcat tgcgcacaca ccagaatcct 2760
   actgagtttg agtattatgg cattgggaaa actgtttttc ttgtaccatt tgttgtgctt 2820
   gtaatttact gtgtttttta ttcggttttc gctatcgaac tgtgaaatgg aaatggatgg 2880
   agaagagtta atgaatgata tggtcctttt gttcattctc aaattaatat tatttgtttt 2940
   ttctcttatt tgttgtgtgt tgaatttgaa attataagag atatgcaaac attttgtttt 3000
   gagtaaaaat gtgtcaaatc gtggcctcta atgaccgaag ttaatatgag gagtaaaaca 3060
   cttgtagttg taccattatg cttattcact aggcaacaaa tatattttca gacctagaaa 3120
   agctgcaaat gttactgaat acaagtatgt cctcttgtgt tttagacatt tatgaacttt 3180
   cctttatgta attttccaga atccttgtca gattctaatc attgctttat aattatagtt 3240
   atactcatgg atttgtagtt gagtatgaaa atatttttta atgcatttta tgacttgcca 3300
   attgattgac aacatgcatc aatcgacctg cagccactcg aagcggccgc cactcgagtg 3360
   gtggccgcat cgatcgtgaa gtttctcatc taagccccca tttggacgtg aatgtagaca 3420
   cgtcgaaata aagatttccg aattagaata atttgtttat tgctttcgcc tataaatacg 3480
   acggatcgta atttgtcgtt ttatcaaaat gtactttcat tttataataa cgctgcggac 3540
   atctacattt ttgaattgaa aaaaattggt aattactctt tctttttctc catattgacc 3600
   atcatactca ttgctgatcc atgtagattt cccggacatg aagccattta caattgaata 3660
   tatcctaagt aaaacctcat aggttttacg tatttcattt agggac 3706
<210> 7
   <211> 23
   <212> DNA
   <213> Artificial Sequence
<220>
   <223> Primer
<400> 7
   atgcattggc ctttgttttt gat 23
<210> 8
   <211> 19
   <212> DNA
   <213> Artificial Sequence
<220>
   <223> Primer
<400> 8
   tgtcgtttcc cgccttcag 19
<210> 9
   <211> 26
   <212> DNA
   <213> Artificial Sequence
<220>
   <223> Primer
<400> 9
   cgctgcggac atctacattt ttgaat 26
<210> 10
   <211> 28
   <212> DNA
   <213> Artificial Sequence
<220>
   <223> Primer
<400> 10
   agttaacttt ccacttatcg gggcactg 28
<210> 11
   <211> 288
   <212> DNA
   <213> Artificial Sequence
<220>
   <223> PCR product
<400> 11
   atgcattggc ctttgttttt gatggcatca actttggagc atctgatttt gcatattcag 60
   ccttttccat ggtaattctt ttacaagaat tttcattctt tcttaagtat aaacacttag 120
   cttgggacaa acttctgatc ctatttctta atttttgcag gcgatggtgg ctgttatgag 180
   cattttgtgt ttgatgtttc tctcttctca ttacggtttt attgggatct gggtggctct 240
   aactatttac atgagcctcc gcgcgtttgc tgaaggcggg aaacgaca 288
<210> 12
   <211> 751
   <212> DNA
   <213> Artificial Sequence
<220>
   <223> PCR product
<400> 12
   cgctgcggac atctacattt ttgaattgaa aaaaaattgg taattactct ttctttttct      60
   ccatattgac catcatactc attgctgatc catgtagatt tcccggacat gaagccattt     120
   acaattgaat atatcctaag taaaacctca taggttttac gtatttcatt tagggactaa     180
   aatggtttag gataattact ttagctaaca taagataata aataaataaa taaataaaaa     240
   taaaatggtt gtagataaat aaggaaatca ataatgaata tgagtgtgag tgataggacg     300
   ggaatgggaa acttttacac tactttaacg ctattgaacg agtatgagta tgttataaac     360
   gtaaaatgtt ttatgtgtta gacaatggcc tcaagtgaaa gtgaccctat taatggagga     420
   aatgcaaacc acgagtctga ggtcacgctc gaagaaatga gggcaaggat cgacgcattg     480
   cgtagcgacc ctgtttttgg agatgccacg ggagatgcta gtgataaccg aatggattta     540
   atgaggttga tgatgatgga gcttttacaa ggaaatcgac aaaggcctag aactgaacaa     600
   gaagagtgct caaacatgtt caagaggttt tcggctcata agcccccaac ttatgatgga     660
   aagccagacc ccactgagtt tgaagaatgg ctcaacggca tggaaaaatt gttcgatgcc     720
   acccagtgcc ccgataagtg gaaagttaac t                                    751
<210> 13
   <211> 664
   <212> DNA
   <213> Artificial Sequence
<220>
   <223> PCR product
<400> 13
   ttaatttttg caggcgatgg tggctgttat gagcattttg tgtttgatgt ttctctcttc      60
   tcattacggt tttattggga tctgggtggc tctaactatt tacatgagcc tccgcgcgtt     120
   tgctgaaggc gggaaacgac aatctgatcc ccatcaagct tgagctcagg atttagcagc 180
   attccagatt gggttcaatc aacaaggtac gagccatatc actttattca aattggtatc 240
   gccaaaacca agaaggaact cccatcctca aaggtttgta aggaagaatt ctcagtccaa 300
   agcctcaaca aggtcagggt acagagtctc caaaccatta gccaaaagct acaggagatc 360
   aatgaagaat cttcaatcaa agtaaactac tgttccagca catgcatcat ggtcagtaag 420
   tttcagaaaa agacatccac cgaagactta aagttagtgg gcatctttga aagtaatctt 480
   gtcaacatcg agcagctggc ttgtggggac cagacaaaaa aggaatggtg cagaattgtt 540
   aggcgcacct accaaaagca tctttgcctt tattgcaaag ataaagcaga ttcctctagt 600
   acaagtgggg aacaaaataa cgtggaaaag agctgtcctg acagcccact cactaatgcg 660
   tatg 664
<210> 14
   <211> 30
   <212> DNA
   <213> Artificial Sequence
<220>
   <223> Primer
<400> 14
   gctctgacac aaccggtaaa tgcattggcc 30
<210> 15
   <211> 30
   <212> DNA
   <213> Artificial Sequence
<220>
   <223> Primer
<400> 15
   gacccatagt ttgattttaa gcacgacatg 30
<210> 16
   <211> 29
   <212> DNA
   <213> Artificial Sequence
<220>
   <223> Primer
<400> 16
   gcagattctg ctaacttgcg ccatcggag 29
<210> 17
   <211> 1042
   <212> DNA
   <213> Artificial Sequence
<220>
   <223> PCR product
<220>
   <221> misc_feature
<223> PCR product
<400> 17
   gctctgacac aaccggtaaa tgcattggcc tttgtttttg atggcatcaa ctttggagca 60
   tctgattttg catattcagc cttttccatg gtaattcttt tacaagaatt ttcattcttt 120
   cttaagtata aacacttagc ttgggacaaa cttctgatcc tatttcttaa tttttgcagg 180
   cgatggtggc tgttatgagc attttgtgtt tgatgtttct ctcttctcat tacggtttta 240
   ttgggatctg ggtggctcta actatttaca tgagcctccg cgcgtttgct gaaggcggga 300
   aacgacaatc tgatccccat caagcttgag ctcaggattt agcagcattc cagattgggt 360
   tcaatcaaca aggtacgagc catatcactt tattcaaatt ggtatcgcca aaaccaagaa 420
   ggaactccca tcctcaaagg tttgtaagga agaattctca gtccaaagcc tcaacaaggt 480
   cagggtacag agtctccaaa ccattagcca aaagctacag gagatcaatg aagaatcttc 540
   aatcaaagta aactactgtt ccagcacatg catcatggtc agtaagtttc agaaaaagac 600
   atccaccgaa gacttaaagt tagtgggcat ctttgaaagt aatcttgtca acatcgagca 660
   gctggcttgt ggggaccaga caaaaaagga atggtgcaga attgttaggc gcacctacca 720
   aaagcatctt tgcctttatt gcaaagataa agcagattcc tctagtacaa gtggggaaca 780
   aaataacgtg gaaaagagct gtcctgacag cccactcact aatgcgtatg acgaacgcag 840
   tgacgaccac aaaagaattc cctctatata agaaggcatt cattcccatt tgaaggatca 900
   tcagatactg aaccaatcct tctagaagat ctaagcttat cgataagctt gatgtaattg 960
   gaggaagatc aaaattttca atccccattc ttcgattgct tcaattgaag tttctccgat 1020
   ggcgcaagtt agcagaatct gc 1042
<210> 18
   <211> 23
   <212> DNA
   <213> Artificial Sequence
<220>
   <223> Primer
<400> 18
   cggtaaatgc attggccttt gtt 23
<210> 19
   <211> 26
   <212> DNA
   <213> Artificial Sequence
<220>
   <223> Primer
<400> 19
   cacccagatc ccaataaaac cgtaat 26
<210> 20
   <211> 28
   <212> DNA
   <213> Artificial Sequence
<220>
   <223> Primer
<400> 20
   aaatggttgt agataaataa ggaaatca 28
<210> 21
   <211> 23
   <212> DNA
   <213> Artificial Sequence
<220>
   <223> Primer
<400> 21
   acatgtttga gcactcttct tgt 23

## Beschreibung

Die Erfindung betrifft Glyphosat-tolerante Zuckerrübenpflanzen, Pflanzenmaterial und Samen sowie ein Verfahren und einen Test-Kit zur Identifitierung einer Glyphosat-toleranten Zuckerrübenpflanze

Die Zuckerrübe (*Beta vulgaris*) wird in vielen Ländern als kommerziell genutzte Kulturpflanze angebaut, mit einem Gesamtertrag von über 240 Millionen metrischen Tonnen.

N-(Phosphonomethyl)-Glycin, allgemein als Glyphosat bezeichnet, ist ein Breitband-Fierbizid, das aufgrund seiner hohen Wirksamkeit, biologischen Abbaubarkeit und geringen Toxizität gegenüber Tieren und Menschen breite Anwendung gefunden hat. Glyphosat hemmt den Shikimisäure-Weg, der zur Synthese von aromatischen Verbindungen, ein,schließlich Aminosäuren und Vitaminen, führt. Insbesondere hemmt Glyphosat die Umsetzung von Phosphoenolbrenztraubensäure und Shikimat-3-phosphat zu 5-Enolpyruvyl-Shikimat-3-phosphat durch Hemmung des Enzyms 5-Enolpyruvyl-Shikimat-3-phosphat-Synthase (EPSP-Synthase oder EPSPS). Wenn konventionelle Pflanzen mit Glyphosat behandelt werden, können die Pflanzen die zum Wachstum und Überleben erforderlichen aromatischen Aminosäuren (z. B. Phenylalanin und Tyrosin) nicht herstellen. EPSPS ist in allen Pflanzen, Bakterien und Pilzen vorhanden. In Tieren, die ihre aromatischen Aminosäuren nicht selbst synthetisieren, kommt es nicht vor. Da der Biosynthese-Weg für aromatische Aminosäuren in Säugetieren, Vögeln oder aquatischen Lebensformen nicht vorhanden ist, besitzt Glyphosat, wenn überhaupt, eine nur geringe Toxizität für diese Organismen. Das EPSPS-Enzym ist natürlicherweise in Nahrungsmitteln von pflanzlicher und mikrobieller Herkunft enthalten.

Glyphosat ist der wirksame Bestandteil von Herbiziden wie beispielsweise Roundup^{®}, hergestellt durch Monsanto Company, USA. Üblicherweise wird es als wasserlösliches Salz wie beispielsweise ein Ammonium-, Alkylamin-, Alkalimetall- oder Trimethylsulfoniumsalz formuliert. Eine der gebräuchlichsten Formulierungen ist das Isopropylaminsalz von Glyphosat, welches die in dem Roundup^{®}-Herbizid eingesetzte Form ist.

Es ist gezeigt worden, daß Glyphosat-tolerante Pflanzen hergestellt werden können, indem man die Fähigkeit, eine Glyphosat-tolerante EPSP-Synthase, z. B. die CP4-EPSPS aus Agrobakterium sp. Stamm CP4AcO, zu bilden, in das Genom der Pflanze einbaut.

Eine Glyphosat-tolerante Zuckerrübenpflanzen kann durch Agrobakterium-vermittelte Transformation, bei der ein Gen, das für eine Glyphosat-tolerante EPSPS wie CP4-EPSPS kodiert, in das Pflanzengenom eingeführt wird, hergestellt werden. Solch eine Zuckerrübenpflanze, die CP4-EPSPS exprimiert, ist in der WO 99/23232 beschrieben worden. Zuckerrübenpflanzen, die aus Zellen angezogen wurden, die mit dem Gen für CP4-EPSPS in dieser Weise transformiert wurden, unterscheiden sich jedoch weitgehend in ihren Eigenschaften, was auf die Tatsache zurückzuführen ist, daß das Gen an einer zufälligen Stelle in das Pflanzengenom inseriert wird. Die Insertion eines bestimmten Transgens an einem bestimmten Ort auf einem Chromosomen wird oftmals als "Event" bezeichnet. Der Begriff "Event" wird auch verwendet, um gentechnisch veränderte Anbaupflanzensorten zu unterscheiden. Erwünschte Events sind sehr selten. Die meisten Events werden verworfen, weil das Transgen in ein für das Wachstum wichtiges Pflanzengen mit der Folge eingebaut wird, daß dieses unterbrochen und nicht exprimiert wird, oder das Transgen in einen Abschnitt des Chromosoms eingebaut wurde, der eine Expression des Transgens nicht oder eine zu schwache Expression zuläßt. Aus diesem Grunde ist es erforderlich, eine große Anzahl von Events zu überprüfen, um ein Event zu identifizieren, das durch eine ausreichende Expression des eingeführten Gen gekennzeichnet ist. Dieses Verfahren ist sehr zeit- und kostenaufwendig, und es ist in keiner Weise garantiert, daß eine Pflanze mit zufriedenstellenden Eigenschaften gefunden wird.

Es ist daher die Aufgabe der vorliegenden Erfindung, eine Zuckerrübenpflanze bereitzustellen, die ein hohes Toleranzniveau gegenüber Glyphosat zeigt, aber keine Nachteile in bezug auf andere wichtige agronomische Eigenschaften wie beispielsweise Wachstum, Ertrag, Qualität, Pathogenresistenz etc. aufweist.

Die erfindungsgemäße Glyphosat-tolerante Zuckerrübenpflanze ist dadurch gekennzeichnet, daß
a) ein DNA-Fragment von der genomischen DNA der Zuckerrübenpflanze, Teilen oder Samen davon, durch Polymerasekettenreaktion mit einem ersten Primer, der die Nukleotidsequenz der SEQ ID NO: 3 aufweist, und einem zweiten Primer, der die Nukleotidsequenz der SEQ ID NO: 4 aufweist, amplifiziert werden kann, wobei das DNA-Fragment mindestens 95 % Identität mit der Nukleotidsequenz der SEQ ID NO: 6 aufweist, und/oder
b) ein DNA-Fragment von der genomischen DNA der Zuckerrübenpflanze, Teilen oder Samen davon, durch Polymerasekettenreaktion mit einem ersten Primer, der die Nukleotidsequenz der SEQ ID NO: 9 aufweist, und einem zweiten Primer, der die Nukleotidsequenz der SEQ ID NO: 10 aufweist, amplifiziert werden kann, wobei das DNA-Fragment mindestens 95 % Identität
   mit der Nukleotidsequenz der SEQ ID NO: 12 aufweist, und/oder
c) ein DNA-Fragment von der genomischen DNA der Zuckerrübenpflanze, Teilen oder Samen davon, durch Polymerasekettenreaktion mit einem ersten Primer, der die Nukleotidsequenz der SEQ ID NO: 14 aufweist, und einem zweiten Primer, der die Nukleotidsequenz der SEQ ID NO: 16 aufweist, amplifiziert werden kann, wobei das DNA-Fragment mindestens 95 % Identität mit der Nukleotidsequenz der SEQ ID NO: 17 aufweist.

Die Polymerasekettenreaktion (PCR) ist eine gut bekannte Standardmethode, die zur Amplifizierung von Nukleinsäuremdlekülen verwendet wird (siehe zum Beispiel US 4,683,202).

Die erfindungsgemäße Zuckerrübenpflanze (im folgenden als "Event H7-1" bezeichnet) weist eine hohe Toleranz gegenüber Glyphosat-Herbiziden auf. Darüber hinaus sind die Wachstumseigenschaften und andere agronomische Eigenschaften des Events H7-1 durch den Transformationsvorgang nicht beeinträchtigt. Event H7-1 exprimiert das Agrobakterium-CP4-EPSPS-Gen, das stabil in das Pflanzengenom integriert ist und der Pflanze Glyphosat-Toleranz vermittelt, auf hohem Niveau. Die Pflanze wurde hergestellt durch Agrobakterium-vermittelte Transformationstechnik unter Verwendung des binären Vektors PV-BVGT08. Dieser Vektor enthielt zwischen der linken und rechten Grenzregion ("Border"-Region) die folgenden Sequenzen: eine kodierende Region, bestehend aus einer für ein Chloroplasten-Transitpeptid kodierenden Sequenz von der EPSPS aus Arabidopsis thaliana (bezeichnet als ctp2), angefügt an die kodierende Sequenz der CP4-EPSPS und unter der Regulation des Braunwurz-Mosaik-Virus(Figwort mosaic virus)-Promotors (pFMV) und der E9-3'-Transkriptionsterminatorsequenz aus Pisum sativum.

In einer bevorzugten Ausführungsform der Erfindung weisen die 3706-bp-, 751-bp-und 1042-bp-DNA-Fragmente mindestens 95%, bevorzugt mindestens 99%, besonders bevorzugt mindestens 99,9 % Identität mit den Nukleotidsequenzen der SEQ ID NO: 6, SEQ ID NO : 12 oder SEQ ID NO: 17 auf. 95% Identität bedeutet beispielsweise, daß 95% der Nukleotide einer gegebenen Sequenz mit der verglichenen Sequenz identisch sind. Zu diesem Zweck können die Sequenzen mit Hilfe des Programms BLAST ausgerichtet und verglichen werden (beispielsweise verfügbar unter http://www.ncbi.nlm.nih.gov/blast/b12seq/b12.html). Am meisten bevorzugt weist das 3706-bp-DNA-Fragment die Nukleotidsequenz der SEQ ID NO: 6, das 751-bp-DNA-Fragment die Nukleotidsequenz der SEQ ID NO: 12 und/oder das 1042-bp-DNA-Fragment die Nukleotidsequenz der SEQ ID NO: 17 auf.

Die vorliegende Erfindung betrifft auch Samen, hinterlegt bei der NCIMB, Aberdeen (Schottland, V.K.), unter der Zugangsnummer NCIMB 41158 oder NCIMB 41159. Solcher Samen kann verwendet werden, um eine Glyphosat-tolerante Zuckerrübenpflanze zu erhalten. Der Samen kann ausgesät werden und die heranwachsende Pflanze wird Glyphosat-tolerant sein.

Die Erfindung betrifft auch eine Zelle, Gewebe oder Teile einer Glyphosat-toleranten Zuckerrübenpflanze.

Ein anderer Aspekt der vorliegenden Erfindung besteht in einem Verfahren zur Identifizierung einer Glyphosat-toleranten Zuckerrübenpflanze, dadurch gekennzeichnet, daß das Verfahren den/die Schritt(e) umfaßt des
a) Amplifizierens eines DNA-Fragments von 3500-3900 bp bevorzugt 3706 bp, von der genomischen DNA der Zuckerrübenpflanze, Teilen oder Samen davon, durch Polymerasekettenreaktion mit einem ersten Primer, der.die Nukleotidsequenz der SEQ ID NO: 3 aufweist, und einem zweiten Primer, der die Nukleotidsequenz der SEQ ID NO: 4 aufweist, und/oder
b) Amplifizierens eines DNA-Fragments von 710-790. bp, bevorzugt 751 bp, von der genomischen DNA der Zuckerrübenpflanze, Teilen oder Samen davon, durch Polymerasekettenreaktion mit einem ersten Primer, der die Nukleotidsequenz der SEQ ID NO: 9 aufweist, und einem zweiten Primer, der die SEQ ID NO: 10 aufweist, und/oder
c) Amplifizierens eines DNA-Fragments von 990-1100 bp, bevorzugt 1042 bp, von der genomischen DNA der Zuckerrübenpflanze durch Polymerasekettenreaktion mit einem ersten Primer, der die Nukleotidsequenz der SEQ ID NO: 14 aufweist, und einem zweiten Primer, der die Nukleotidsequenz der SEQ ID NO: 16 aufweist.

Das Verfahren erlaubt die Detektion einer transgenen Glyphosat-toleranten Zuckerrübenpflanze mit Hilfe von Standardmethoden der Molekularbiologie.

Die Erfindung betrifft darüber hinaus einen Test-Kit zur Identifizierung einer transgenen Glyphosat-toleranten Zuckerrübenpflanze, deren Zellen, Gewebe oder Teile, umfassend mindestens ein Primer-Paar mit einem ersten und einem zweiten Primer für eine Polymerasekettenreaktion, wobei der erste Primer eine Sequenz innerhalb der in das Genom der Pflanze aufgenommenen fremden DNA erkennt, und der zweite Primer eine Sequenz innerhalb der flankierenden 3'- oder 5'-Bereiche der DNA erkennt, wobei die Pflanze eine Pflanze nach Anspruch 1 ist.

Nach einer bevorzuglen Ausgestaltung der Erfindung ist der Test-Kit dadurch gekennzeichnet, daβß
a) der erste Primer die Nukleotädsequenz der SEQ ID NO: 9 aufweist und der zweite Primer die Nukleotidsequenz der SEQ ID NO: 10 aufweist, und/oder
b) der erste Primer die Nukleotidsequenz der SEQ ID NO: 14 aufweist und der zweite Primer die Nukleotidsequenz der SEQ ID NO: 16 aufweist.

Bevorzugt weist der erste Primer die Nukleotidsequenz der SEQ ID NO: 9 oder SEQ ID NO : 14 auf, und der zweite Primer weist bevorzugt die Nukleo-tidsequenz der SEQ ID NO: 10 oder SEQ ID NO: 16 auf.

Die folgenden Figuren dienen zur Erläuterung der Erfindung. Es zeigt:
Fig. 1 Eine Karte des binären Vektors und PV-BVGT08.
Fig. 2 Die Identifizierung von H7-1 durch PCR-Analyse. DNA-Proben von 18 Pflanzen wurden analysiert. Neg.-Kontrolle = DNA aus nicht-transformierter Zuckerrübe; Pos.-Kontrolle = DNA aus der Original-H7-1-Transformante.
Fig. 3 Die Identifizierung von Event H7-1 durch Multiplex-PCR und Unterscheidung zwischen transgenem Event H7-1 und nicht-transgenen Pflanzen. DNA-Proben aus 54 Pflanzen wurden analysiert.
Fig. 4 Das pFMV-ctp2-CP4-EPSPS-E9-3'-Insert mit Schnittstellen der Restriktionsenzyme HindIII, XbaI, ClaI, PstI und BamHI.
Fig. 5 Insert-/Kopienzahl-Analyse für Event H7-1. Für die Southern-Blot-Analyse wurden 10 µg genomische DNA von H7-1 mit PstI, HindIII, XbaI, ClaI und BamHI verdaut (Spur 3 bis 7). Nicht-transformierte genomische DNA als Negativkontrolle wurde mit BamHI verdaut (Spur 8). Plasmid PV-BVGT08 als Positivkontrolle wurde mit BamHI verdaut. Die Spuren 2 und 9 repräsentieren Größenmarker. Der Blot wurde mit einer ³²P-markierten kodierenden Regionen von CP4-EPSPS sondiert. Die Sonde ist eine interne Sequenz des CP4-EPSPS-Gens, umfassend die Basenpaare 447 bis 1555.
Fig. 6 Southern-Blot-Analyse von H7-1 zur Ermittlung der Intaktheit der kodierenden Region der ctp2-CP4-EPSPS. 10 µg genomische DNA von H7-1, nicht-transgene Kontroll-DNA und nicht-transgene Kontroll-DNA, gemischt mit PV-BVGT08, wurden mit XbaI und HindIII/BamHI verdaut. Der Blot wurde mit einem ³²P-markierten CP4-EPSPS-PCR-Fragment sondiert.
Fig. 7 Southern-Blot-Analyse von Event H7-1 zur Ermittlung der.Intaktheit der Promotorregion. 10 µg genomische DNA von H7-1, nicht-transgene Kontroll-DNA und nicht-transgene Kontroll-DNA, gemischt mit PV-BVGT08; wurden mit HindIII, XbaI und SacI/XhoI verdaut. Der Blot wurde mit einem ³²P-markierte Promotor-Fragment (HindIII) (=PV-HVGT08-Sequenz, Bp 7972-8583) oder der vollständigen Promotor-ctp2-CP4-EPSPS-E9-3'-Kassette (PmeI/XhoI) (=PV-BVGT08-Sequenz, Bp 7935-2389) sondiert.
Fig. 8 Southern-Blot-Analyse von Event H7-1 zur Ermittlung der Intaktheit der polyadenylierten(Poly-A-)Region. 10 µg genomische DNA von H7-1, nicht-transgene Kontroll-DNA und nicht-transgene Kontroll-DNA, gemischt mit PV-BVGT08, wurden mit EcoRI/PstI, XbaI, HindIII und PstI verdaut. Der Blot wurde mit einem ³²P-markierten Poly-A-Fragment (*BamHI*/*XhoI*) (mPV-BVGT08-Sequenz, Bp 1702-2389) sondiert.
Fig. 9. Als Sonden zur Feststellung der Abwesenheit von "Hackbone"-Vektor-DNA in Event H7-1 verwendete Fragmente.
Fig. 10 Southern-Blot-Analyse von Event H7-1 zur Feststellung der Abwesenheit von "Backbone"-DNA in Event H7-1. 10 µg genomische DNA von H7-1, nicht-transgene Kontroll-DNA und nicht-transgene Kontroll-DNA, gemischt mit PV-BVGT08, wurden mit XbaI verdaut. Der Blot wurde mit einem ³²P-markierten Fragment, das das vollständige "Backbone" von PV-BVGT08 (Sonde 1-4) umfaßt, sondiert.
Fig. 11 Vergleich zwischen den PCR-Fragmenten und den PV-BVGT08-Sequenzen in der linken Grenzregion (left border, LB).
Fig. 12 Vergleich zwischen den PCR-Fragmenten und den PV-BVGT08-Sequenzen in der rechten Grenzregion (right border, RB).
Fig. 13 Analyse der genomischen DNA außerhalb der rechten Verbindungsstelle des Inserts. Ungefähr jeweils 50 ng genomische DNA von H7-1, nicht-transgene Kontroll-DNA oder Wasser wurden für PCR-Reaktionen mit der Primer-Kombinationen P1, bei der beide Primer außerhalb des Inserts lokalisiert sind, und P3, bei der ein Primer innerhalb des Inserts lokalisiert ist und der andere Primer außerhalb des Insert liegt, verwendet.
Fig. 14 Analyse der genomischen DNA außerhalb der linken Verbindungsstelle des Inserts. Ungefähre50 ng genomische DNA von H7-1, nicht-transgene Kontroll-DNA und Wasser wurden für PCR-Reaktionen mit der Primer-Kombinationen P2, bei der beide Primer außerhalb des Inserts lokalisiert sind, und P4, bei der ein Primer innerhalb des Inserts lokalisiert ist und der andere Primer außerhalb des Insert liegt, verwendet.
Fig. 15 Abstammungskarte zu H7-1 Saatkulturen.
Fig. 16 Southern-Blot-Analyse von Event H7-1, um zu ermitteln, ob die eingefügte DNA stabil in das Genom integriert ist. 10 µg genomische DNA von H7-1 (der ursprüngliche Transformant H-7-1-1995 und drei Nachkommen, H7-1-1996 bis 1998) und nicht-transgene Kontroll-DNAs von verschiedener Herkunft wurden mit BamHI, XbaI und HindIII verdaut. Der Blot wurde mit einer ³²P-markierten CP4-EPSPS-Sonde von PV-BVGT08 (= Bp 447-1555) untersucht.

Die Erfindung wird nachfolgend anhand von ausgewählten Beispielen näher erläutern.

Eine Liste von Abkürzungen ist unten wiedergegeben:
- -: ungefähr
- °C: Grad Celsius
- bidest: doppelt destilliertes steriles Wasser
- bp: Basenpaar (e)
- CTAB: Cetyltrimethylammoniumbromid
- DNA: Desoxyribonukleinsäure
- E. coli: Escherichia coli
- EDTA: Ethylendiamintetraessigsäure
- Fig.: Figur
- h: Stunde
- IiCl: Salzsäure
- kb: Kilobasenpaar(e)
- kg: Kilogramm
- M, mM,: molar, millimolar
- min: Minuten
- Na₂HPO₄/NaH₂PO₄: Natriumphosphat
- NaCl: Natriumchlorid
- NaOH: Natriumhydroxid
- nt: Nukleotide
- PCR: Polymerasekettenreaktion
- pmol: Picomol
- RNase: Ribonukleinsäure-Nuklease
- UPM: Umdrehungen pro Minute
- RR: Roundup Ready^{®}
- RT: Raumtemperatur
- SDS: Natriumdodecylsulfat
- sek: Sekunde
- SEVAG: Chloroform : Isoamylalkohol (24 : 1)
- SSC: standard saline citrate
- TE: Tris-EDTA-Puffer
- TRIS: Tris(hydroxymethyl)-aminomethan

### BEISPIEL 1: Identifizierung von Event H7-1

Die Zuckerrübe (Beta vulgaris) vom Genotyp 350057 ist in einer Weise genetisch verändert, daß sie eine CP4-5-Enolpyruvyl-Shikimat-3-phosphat-Synthase oder CP4-EPSPS exprimiert, die Toleranz gegenüber dem Herbizid Glyphosat vermittelt und auch als selektierbarer Marker verwendet wird. Diese transgene Linie wurde durch Agrobakterium-tumefaciensvermittelte Transformationstechnologie unter Verwendung des binären Vektors PV-BVGT08 hergestellt. Die T-DNA des für die Zuckerrüben-Transformation verwendeten Vektors enthielt zwischen der linken und der rechten Grenzregion die folgenden Sequenzen: eine kodierende Region, zusammengesetzt aus einer für ein Chloroplasten-Transitpeptid kodierenden Sequenz aus der EPSPS aus Arabidopsis-thaliana (bezeichnet als ctp2), fusioniert mit der kodierenden Sequenz für CP4-EPSPS und unter Regulation des 35S-Braunwurz-Mosaik-Virus(Figwort mosaic virus)-Promotors (pFMV) und der 3'-Transkriptionsterminatorsequenz des rbcS-E9-Gens aus Pisum sativum.

Die folgenden Methoden wurden eingesetzt:

### I. DNA-Extraktion:

### Methode 1:

Frisches Blatt- oder anderes Gewebe wurde gesammelt (20 bis 100 mg in einem 1,5-ml-Reaktionsgefäß) und 400 µl Extraktions-Puffer (siehe unten) wurden zugegeben. Das Gewebe wurde mit einem kleinen Stößel zermahlen. Die Mischung wurde für 5 sek verwirbelt und 30 min bis 60 min bei Raumtemperatur inkubiert. Es folgte ein Zentrifugations-Schritt bei 13.000 UPM für 1 min. Der die DNA enthaltende Überstand wurde in ein neues 1,5-ml-.Reaktionsgefäß gegossen und mit 120 µl Isopropanol gemischt. Die Mischung wurde bei Raumtemperatur für 2 min inkubiert. Ein DNA-Niederschlag wird nach Zugabe von Ethanol gebildet. Nach Zentrifugation bei 13.000 UPM für 5 Minuten wurde das Ethanol dekantiert. Die Probe wurde an der Luft trocknen gelassen. Der Niederschlag wurde in 400 µl H₂O oder TE-Puffer (sie unten) resuspendiert.

### Extraktions-Puffer (100 ml):

| | |
|---|---|
| 20 ml | 1 M Tris (pH 7,5) |
| 5 ml | 5 M NaCl |
| 5 ml | 0,5 M EDTA |
| 2,5 ml | 20% SDS |
| 67,5 ml | H₂0 |

### TE-Puffer:

10 mM Tris-HCl (pH 8,0)
1 mM EDTA

### Methode 2:

Frisches Pflanzenmaterial (20 bis 100 mg) wurde in einem 1,5-ml-Eppendorfgefäß gesammelt und 500 µl CTAB-Puffer (65 °C) (siehe unten) wurden zugegeben. Die Mischung wurde 1 bis 1,5 h bei 65 °C inkubiert und anschließend für 5 sek zentrifugiert. 5 µl RNase A (10 mg/ml) wurden zugegeben. Die Mischung wurde 30 min bei 37 °C inkubiert und anschließend für 5 sek zentrifugiert. 200 µl SEVAG wurden zugefügt. Nach Mischen und Zentrifugieren bei 13.000 UPM für 10 min wurde der Überstand in ein neues 1,5-ml-Reaktionsgefäß überführt. Ein Volumen Isopropanol (etwa 400 µl) wurde vorsichtig mit dem Überstand vermischt. Es folgte ein Zentrifugationsschritt bei 13.000 UPM für 10 min. 600 µl 70% Ethanol wurden zugefügt. Der Niederschlag wurde durch Invertieren des Reaktionsgefäßes mehrere Male gewaschen. Erneut wurde die Mischung bei 13.000 UPM für 2 min gewaschen. Der Ethanol wurde vorsichtig verworfen. Das Reaktionsgefäß wurde invertiert und auf sauberem Papier drainiert. Die Probe wurde für 15 min an der Luft getrocknet. Der Niederschlag wurde in 50 µl H₂O (siehe unten) resuspendiert.

### CTAB-Puffer:

1,4 M NaCl
20 mM EDTA
100 mM Tris-HCl
2 % (w/v) CTAB

### SEVAG:

Chloroform : Isoamylalkohol (24 : 1)

### RNase-Puffer:

10 mM Tris, 15 mM NaCl, pH 7,5

### RNase A:

10 mg RNase/ml RNase-Puffer (5 ml bidest + 50 mg RNase A, Aliquots in 1,5-ml-Reaktionsgefäßen, koche die Reaktionsgefäße 30 min bei 100°C, Lagerung bei -20°C)

Normalerweise wurde Methode 1 verwendet. Mit dieser Methode ist es möglich, eine hohe Zahl von DNA-Proben pro Tag zu extrahieren, und die DNA-Qualität ist akzeptabel. Methode 2 wurde angewendet, wenn das Blattmaterial älter war oder Probleme mit der DNA-Qualität auftraten. Methode 2 ist komplexer, erfordert mehr Zeit und ergibt einen geringeren DNA-Ertrag, jedoch von höherer Qualität.

Normalerweise wird eine DNA-Quantifizierung nicht durchgeführt und typischerweise werden in einer PCR-Reaktion 0,5 bis 1 µl der extrahierten DNA-Lösung verwendet.

### II. PCR-Reaktion:

Für die PCR-Reaktion wurde ein 10x Puffer-Mix aus Puffer + dNTPs hergestellt. Das Verfahren ist wie folgt:

| Stammlösung | Volumen | Konz. 10x-Puffer | Endkonz. PCR-Reaktion |
|---|---|---|---|
| 1 M Tris-HCl, pH 8,3 | 100 µl | 0,1 M | 10 mM Tris-HCl, pH 8,3 |
| 1 M KCl | 500 µl | 0,5 M | 50 mM KCl |
| 100 mM dATP | 20 µl | 2 mM | 0,2 mM dATP |
| 100 mM dCTP | 20 µl | 2 mM | 0,2 mM dCTP |
| 100 mM dTTP | 20 µl | 2 mM | 0,2 mM dTTP |
| 100 mM dGTP | 20 µl | 2 mM | 0,2 mM dGTP |
| 100 mM MgCl₂ | 150 µl | 15 mM | 1,5 mM MgCl₂ |
| HPLC-Wasser | 170 µl | | |
| | 1000 µl | gesamt | |

### PCR-Reaktion (25 µl):

| | |
|---|---|
| DNA | 0,5 µl |
| Primer 1 | 1 µl (20 pmol) |
| Primer 2 | 1 µl (20 pmol) |
| Taq-Polymerase 1 | 0,2 µl (1 U, von Oncor Appligene S.A., Heidelberg, Deutschland) |
| Puffer 10x konz. | 2,5 µl |
| Wasser | 18,8 µl |

### III. Identifizierung von H7-1 durch PCR

Die Identifizierung von H7-1 wurde mittels PCR unter Verwendung von Event-spezifischen Primern durchgeführt:

### Oberer Primer (SEQ ID NO: 1):

H7-207U30: 5' TTA ATT TTT GCA GGC GAT GGT .GGC TGT TAT 3'

### Unterer Primer (SEQ ID NO: 2):

H7-841L30: 5' CAT ACG CAT TAG TGA GTG GGC TGT CAG GAC 3'

Der obere Primer ist außerhalb des Inserts lokalisiert und ist Teil der genomischen DNA der Zuckerrübe. Der unterer Primer ist innerhalb des eingefügten CP4-EPSPS-Gens lokalisiert.

### PCR-Bedingungen:

| | |
|---|---|
| 94°C 4 min | SCHRITT 1 |
| 95°C 30 sec | SCHRITT 2a |
| 55°C 30 sec | SCHRITT 2b |
| 72°C 2 min | SCHRITT 2c |
| 72°C 5 min | SCHRITT 3 |
| 4°C über Nacht | SCHRITT 4 |
| | Reaktion beendet |

Die Schritte 2a-c wurden 34mal wiederholt.

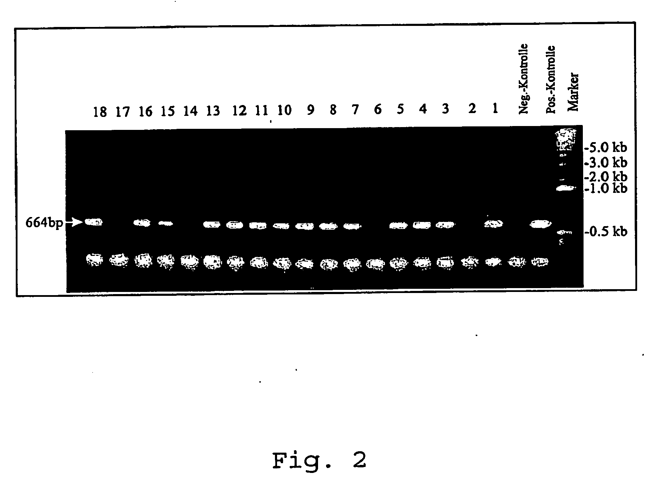

Das erwartete PCR-Produkt ist ein DNA-Fragment von 664 bp (SEQ ID NO: 13, siehe Fig. 2).

### IV. Identifizierung von H7-1 durch Multiplex-PCR

Zur Unterscheidung von nicht-transgenen und transgenen Pflanzen, ob homozygot oder heterozygot, wurde eine Multiplex-PCR mit drei verschiedenen Primern durchgeführt. Die folgenden Primer wurden verwendet:
H72 (SEQ ID NO: 14): 5' GCTCTGACACAACCGGTAAATGCATTGGCC 3'
H7S2 (SEQ ID NO: 15): 5' GACCCATAGTTTGATTTTAAGCACGACATG 3'
H7R2 (SEQ ID NO: 16): 5' GCAGATTCTGCTAACTTGCGCCATCGGAG 3'

### PCR-Bedingungen:

| | |
|---|---|
| 94°C 2 min | SCHRITT 1 |
| 94°C 1 sec | SCHRITT 2a |
| 60°C 45 sec | SCHRITT 2b |
| 72°C 90 sec | SCHRITT 2c |
| 72°C 5 min | SCHRITT 3 |
| 4°C über Nacht | SCHRITT 4 |
| | Reaktion beendet |

Die Schritte 2a-c wurden 34mal wiederholt.

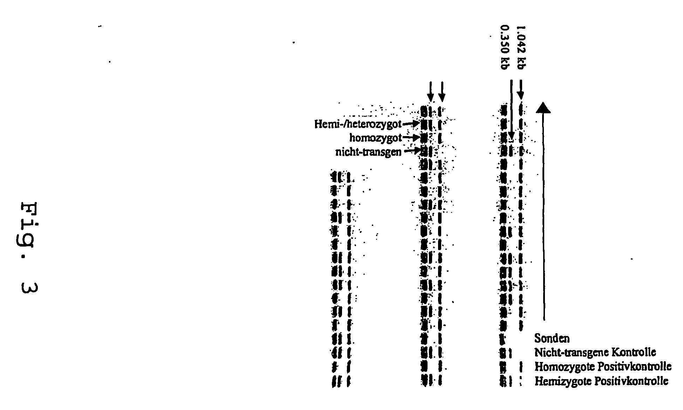

Nicht-transgene Pflanzen zeigten lediglich ein PCR-Fragment von ungefähr 350 bp. Homozygote transgene Pflanzen zeigten ein Fragment von ungefähr 1.042 kb. Heterozygote Pflanzen zeigten beide Fragmente (siehe Fig. 3).

### BEISPIEL 2: Charakterisierung von Event H7-1

Es wurde eine molekulare Analyse zur Charakterisierung der in H7-1 vorhandenen integrierten DNA durchgeführt. Insbesondere wurde die Zahl der Inserts (Zahl der Integrationsstellen innerhalb des Zuckerrübengenoms), die Kopienzahl (die Zahl von DNA-Fragmenten innerhalb eines Ortes), die Integrität der eingefügten kodierenden Region und dessen regulatorischer Elemente, dem pFMV-Promotor und der E9-3'-Transkriptionsterminatorsequenz, die Abwesenheit von "Backbone"-Sequenzen des zur Transformationen verwendeten Vektors und die stabile Vererbung des Inserts bestimmt. Darüber hinaus wurden die das DNA-Insert flankierenden Sequenzen identifiziert.

Die eingefügte DNA des Zuckerrüben-Transformations-Events H7-1 wurde mittels Southern-Blot, PCR und Invers-PCR-Techniken charakterisiert. Positiv- und Negativkontrollen (PV-BVGT08, DNA von nicht-transgenen Pflanzen) wurden mit untersucht und in der gleichen Weise wie die Testsubstanz (H7-1) behandelt.

DNA wurde aus dem Ansatz Nummer 74903H von Event-H7-1-Pflanzen, angezogen im Jahr 1997, isoliert. Auch aus der Original-Transformante H7-1/3S0057 (= 6401VH) von 1995 und aus drei zusätzlichen in den Jahren 1996, 1997 und 1998 hergestellten Nachkommen (H7-1/64801H, H7-1/74922H und H7-1/83002S) wurde DNA isoliert.

Die nicht-transgene Zuckerrübenlinie 3S0057 diente als Kontrolle. Darüber hinaus wurden die Zuckerrübenlinien 5R7150, 8K1180 und 6S0085 als Negativkontrolle eingesetzt. Diese Linien sind gewöhnliche nicht-transgene Linien, die zur Zucht konventioneller Zuckerrüben verwendet werden.

Referenzsubstanzen entsprachen dem zur Transformation verwendeten Plasmid PV-BVGT08. Die Plasmid-DNA und DNA aus den Kontroll-Zuckerrübenlinien wurde zusammengemischt, mit Restriktionsenzymen verdaut und durch Elektrophorese auf Agarose-Gelen parallel zu den Testsubstanzen getrennt. Das Plasmid diente als Größenmarker für das erwartete Fragment und als positive Hybridisierungs-Kontrolle. Die Plasmid-DNA wurde mit der genomischen Pflanzen-DNA in einer Konzentration gemischt, die weniger als eine Kopie des zu analysierenden Elements repräsentiert, um die Empfindlichkeit der Southern-Blot-Methode (-10 µg genomische DNA und -28 pg PV-BVGT08-DNA) zu demonstrieren. Für Größenabschätzungen wurde der Molekülgrößenmarker RAOUL^{™} (ONCOR/Appligene, Katalog #160673) verwendet.

### DNA-Isolierung:

Pflanzengewebe (1 bis 3 g Feuchtgewicht) vom Ansatz Nummer 74903H des Events H7-1 wurden unter Verwendung eines Mörsers und Stößels in flüssigem Stickstoff zu einem feinen Pulver zermahlen. Das Pulver wurde in ein 50-ml-Oakridge-Gefäß transferiert und 7,5 ml vorgewärmter (60 °C) CTAB-Puffer (2 % CTAB, 100 mM Tris-HCl, 20 mM EDTA pH 8,0, 1,4 M NaCl and 0,2 % Mercaptoethanol) wurden zugegeben. Die Proben wurden für ungefähr 30 min bei 65 °C inkubiert und zwischendurch gemischt. Ein gleiches Volumen (8 ml) einer Mischung aus Chloroform : Isoamylalkohol (24:1 v/v), RT, wurde zu den Proben zugegeben. Die Suspension wurde durch Invertieren gemischt und die zwei Phasen wurden durch Zentrifugation (10 min, 9000 UPM) getrennt. Die wäßrige Phase wurde in ein neues 50-ml-Oakridge-Gefäß überführt und anschließend die DNA durch Zugabe von 5 ml Isopropanol gefällt. Die DNA wurde durch Zentrifugation (2 min, 9000 UPM) niedergeschlagen und der Überstand wurde entfernt. Die niedergeschlagene DNA wurde mit einer Waschlösung aus 76% Ethanol und 10 mM Ammoniumacetat für ungefähr 20 min inkubiert. Nach der Zentrifugation und dem Dekantieren des Überstandes wurde die DNA vakuumgetrocknet und in TE, pH 8,0, bei 4 °C über Nacht resuspendiert.

Alternativ wurde DNA mittels des DNeasy Plant Maxi Kits von Qiagen (Düsseldorf, Deutschland, Katalog #68163) isoliert. Die DNA-Isolierung wurde nach den Herstellerangaben durchgeführt.

Als zusätzliche alternative Methode wurde DNA mittels des DNeasy Plant Mini Kits von Qiagen (Düsseldorf, Deutschland, Katalog #69103) isoliert. Die DNA-Isolierung wurde nach den Herstellerangaben durchgeführt.

### Quantifizierung der DNA und Verdauung durch Restriktionsenzyme:

Die Quantifizierung der DNA wurde unter Verwendung eines LKB Biochrom UV/visible Spektralphotometers (Amersham Pharmacia, Freiburg, Deutschland) oder alternativ durch Scannen der DNA mit dem Programm RFLPscan (MWG-Biotech, Ebersberg, Deutschland) nach Agarose-Gelelektrophorese durchgeführt. Als Kalibrierungsstandard wurde der High DNA Mass Ladder von Gibco/Life Technologies (Karlsruhe, Deutschland)(Katalog # 10496-016) verwendet. Die Restriktionsenzyme wurden entweder von Boehringer Mannheim (Mannheim, Deutschland), Stratagene (Amsterdam, Niederlande) oder New England Biolabs (Frankfurt, Deutschland) bezogen und nach Herstellerangaben verwendet.

### DNA-Sonden-Herstellung:

PV-BVGT08-DNA^{.}wurde aus E. coli-Kulturen isoliert. Zu der kodierenden Region der CP4-EPSPS, dem 35S-Promotor, der E9-3'-Poly-A-Region, der 35S-ctp2-CP4-EPSPS-E9-3'-Kassette und den "Hackbone"-Bereichen homologe Sonden-Matrizen wurden durch Verdauung mit den entsprechenden Restriktionsenzymen und anschließende Trennung durch Agarose-Gelelektrophorese oder durch Polymerasekettenreaktion (PCR) hergestellt. Die Produkte wurden mittels des Gene Clean II Kits von BIO 101 (La Jolla, CA) gereinigt. Die Markierung der Sonden (25 pg) mit ³²P-dCTP oder ³²P-dATP wurde durch Verwendung des Megaprime^{™}-DNA-Markierungssystems von Amersham-Pharmacia Biotech Europe (Freiburg, Deutschland) erreicht.

### Southern-Blot-Analyse:

Die mit Restriktionsenzymen behandelten DNA-Proben wurden durch Agarose-Gelelektrophorese für ~15 Stunden bei ~35 Volt getrennt. Nach dem Fotografieren des Gels wurde die DNA durch Tränken des Gels für 15 min in einer 0,25 M HCl-Lösung depuriniert, durch Inkubieren des Gels für 30 min in einer denaturierenden Lösung aus 0,5 M NaOH, 1,5 M NaCl unter konstanter sanfter Bewegung denaturiert und schließlich durch Tränken für 2 Stunden in verschiedenen Volumina einer Lösung aus 2 M NaCl und 1 M Tris-HCl, pH 5,5, neutralisiert. Die DNAs aus den Agarose-Gelen wurden unter Verwendung eines Pressure Blotter von Stratagene nach Anweisungen des Herstellers auf Hybond-N^{™}-Nylonmembranen (Amersham Pharmacia Biotech Europe, Freiburg, Deutschland) übertragen. Nach dem Tränken der Filter für 15 min in 2 x SSPE (20 x SSPE: 3,6 M NaCl, 20 mM EDTA, 0,2 M NaH₂PO₄/Na₂HPO₄ pH 7,4) wurde die DNA durch Belichtung mit W-Licht (Transilluminator Pharmacia, Freibürg, Deutschland) für 1 min und durch Erhitzen bei 80 °C für 1 Stunde in einem Vakuumofen auf der Membran fixiert. Die Blots wurden für 4 Stunden in einer wäßrigen Lösung aus 50 % Formamid, 5 x SSC, 0,1 % Lauroylsarcosin, 0,02 % SDS und 2 % Blockreagenz (Boehringer Mannheim, Deutschland, Katalog # 1096176) vorhybridisiert. Die Hybridisierung mit der radioaktiv-markierten Sonde wurde in frischer Vorhybridisierungs-Lösung für 16-18 Stunden bei 42 °C durchgeführt. Nach der Hybridisierung wurden die Membranen für 5 min in 2 x SSC bei 42°C, für 20 min in 2 x SSC, 1 % SDS bei 65°C und für zwei 15-min-Perioden in 0,2 x SSC, 0,1 % SDS bei 68°C gewaschen. Autoradiographiebilder von den Blots wurden durch Exponieren der Blots unter Verwendung eines Biomax-MS^{™}-Films in Verbindung mit Kodak Biomax MS^{™} Verstärkungsfolien erhalten.

### Identifizierung flankierender 5'- und 3'-Genomsequenzen:

Die Verbindungsstelle zwischen der Transgen- und Pflanzengenom-DNA wurde unter Einsatz der Invers-PCR-Technik identifiziert. Genomische DNA wurde wie oben beschrieben gereinigt. Ungefähr 1 µg der DNA wurde in getrennten Reaktionen durch die Restriktionsendonukleasen TaqI, AluI, NdeIII oder RsaI verdauten. Die verdauten DNA-Fragmente wurden durch T4-Ligase über Nacht religiert, worauf sich die PCR-Reaktion anschloß. Die verschiedenen Invers-Primer-Kombinationen wurden unter Verwendung der Primer-Analysesoftware OLIGO^{®} von NBI (National Biosciences, Inc., Plymouth, Michigan) erhalten.

Aus dieser Invers-PCR-Amplifikation erhaltene Fragmente wurden durch Gel-Elektrophorese getrennt, aus dem Gel ausgeschnitten und unter Verwendung des Gene Clean II^{™} Kits gereinigt. Die gereinigten Fragmente wurden unter Einsatz des TOPO^{™}TA cloning^{®} Kit von Invitrogen (Groningen, Niederlande) in den Vektor pCR^{®} 2.1 kloniert. Die Inserts wurden zur Sequenzierung bei MWG-Biotech (Ebersberg, Deutschland) eingereicht. Die Analyse der erhaltenen Sequenzdaten wurde unter Verwendung der DNA-Analysesoftware Mac Molly^{®} Tetra (Soft Gene GmbH, Bochold, Deutschland) durchgeführt.

### PCR-Analyse:

Genomische DNA wurde mit dem DNAeasy Plant Mini Kit (Qiagen, Düsseldorf, Deutschland) nach Herstellerangaben hergestellt. Ungefähr 50 ng genomische DNA wurden für die PCR verwendet.

Die Reaktionsansätze wurden für 30 sek 95°C, für 30 sek 55°C und für 2 min 72°C für 35 Zyklen ausgesetzt. Die PCR wurde in einem PTC200-Cycler (Biozym, Oldendorf, Deutschland) durchgeführt. Die PCR-Produkte wurden durch Agarose-Gelelektrophorese analysiert.

### 1. Anzahl Inserts:

Die Anzahl der Inserts, die Zahl der Integrationsstellen transgener DNA im Zuckerrübengenom, wurde für H7-1 bestimmt. Um die Zahl der Inserts festzustellen, wurde die genomische DNA mit den Restriktionsenzymen HindIII, XbaI und BamHI verdaut. Als Negativkontrolle wurde DNA aus einer nichttransformierten Kontroll-Pflanze, die denselben genetischen Hintergrund repräsentiert, mit Hind III verdaut. Als Positivkontrolle wurde DNA des Transformationsvektors (PV-BVGT08) verwendet.

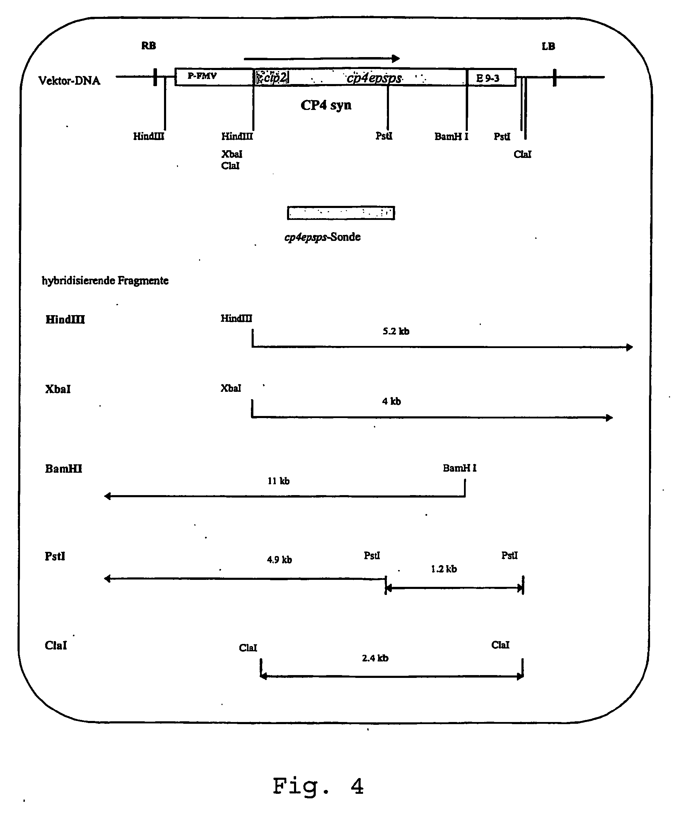

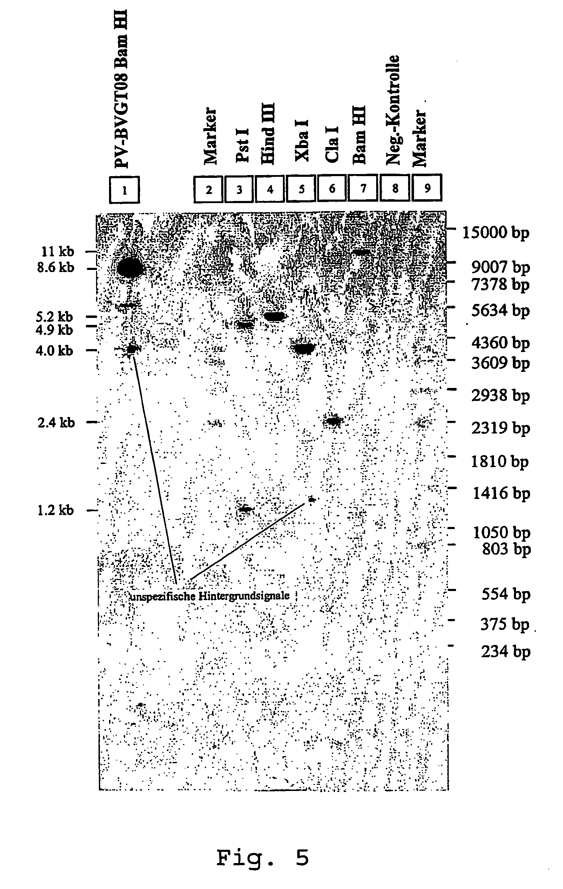

XbaI und BamHI schneiden PV-BVGT08 nur einmal und schneiden nicht innerhalb der verwendeten markierten CP4-EPSPS-Sonde (siehe Fig. 4). HindIII schneidet PV-BVGT08 dreimal, aber alle drei Stellen sind außerhalb der Sonde und auf derselben Seite, 5' relativ zur Sonde, lokalisiert. Daher sollte jedes Enzym ein einzelnes DNA-Fragment freisetzen, das mit der CP4-EPSPS-Sonde hybridisieren und einen Teil der eingefügten DNA sowie angrenzende genomische Pflanzen-DNA enthalten würde. Die Zahl der festgestellten Fragmente zeigt die Zahl der im Event vorhandenen Inserts an. Die Ergebnisse sind in Fig. 5 dargestellt.

Nach Verdauung mit den Enzymen HindIII, XbaI oder BamHI wurde jeweils lediglich ein einzelnes Hybridisierungs-Fragment gefunden. Die Fragmente aus 5,2 kb von der HindIII-Verdauung (Spur 4), 4 kb von der XbaI-Verdauung (Spur 5) und ungefähr 11 kb von der BamHI-Verdauung (Spur 7) zeigen, daß die Transformante H7-1 ein einzelnes Integrationsereignis repräsentiert (Fig. 4). Das starke Signal in Spur 1 repräsentiert das linearisierte PV-BVGT08-Plasmid. Zusätzliche schwache Signale sind auf kleine Mengen von unverdautem PV-BVGT08 oder auf unspezifische Hintergrund-Hybridisierungs-Signale zurückzuführen.

### 2. Kopienzahl

Theoretisch könnte eine Integrationsstelle aus mehr als einer Kopie der eingefügten DNA bestehen. Aufgrund der Fragmentgrößen der oben dargestellten Restriktionsanalyse war dies aber nicht möglich. Wenn mehr als eine Kopie der eingefügten DNA H7-1 vorhanden wäre, würden zusätzliche Fragmente detektiert werden. Dies wurde auch bestätigt durch Verdauung mit dem Restriktionsenzym PstI. PstI schneidet zweimal innerhalb der linken und der rechten Grenzsequenzen. Eine der Restriktionsschnittstellen liegt innerhalb der kodierenden Region der CP4-EPSPS, so daß nach der Verdauung zwei mit der CP4-EPSPS-Sonde hybridisierende Fragmente zu erwarten sind. Eines der erwarteten Fragmente entspricht dem internen Fragment von etwa 1,2 kb. Das zweite Fragment sollte ein Grenzfragment sein. Auch hier müßten zusätzliche Fragmente feststellbar sein, wenn mehr als eine Kopie vorhanden ist. Die Ergebnisse zeigen aber, daß PstI die DNA wie erwartet schneidet. Das innere Fragment von 1,2 kb und nur ein zusätzliches Fragment von ungefähr 4,9 kb wurden detektiert (Fig. 5, Spur 3; Fig. 4).

Als zusätzliche interne Kontrolle wurde die DNA mit ClaI geschnitten (Fig. 5, Spur 6; Fig. 4). Wie erwartet hybridisierte ein einzelnes Fragment von 2,4 kb, da ClaI zweimal, aber links und rechts außerhalb des verwendeten CP4-EPSPS-Fragments schneidet. Dieses Ergebnis ist auch ein Beleg für die Intaktheit des integrierten DNA-Fragments und steht in Übereinstimmung mit den im folgenden wiedergegebenen Ergebnissen.

Die Hybridisierung des Plasmids PV-BVGT08 (PV-BVGT08 = 8590 bp) mit dem CP4-EPSPS-Fragment führt wie erwartet zu einem 8,6-kb-Signal (Fig. 5, Spur 1). Eine zweite kleinere sehr schwache Bande ist auf die unvollständige Restriktion von PV-BVGT08 zurückzuführen.

Zusammengefaßt zeigen die Experimente, daß die transformierte Zuckerrübenlinie H7-1 eine einzige Kopie der T-DNA von PV-BVGT08 im Pflanzengenom enthält.

### 3. Intaktheit der kodierenden Region

Der Einbau der CP4-EPSPS-Genkassette mit den einzelnen Elementen (P-FMV-Promotor, kodierende Region der ctp2-CP4-EPSPS und E9-3'-untranslatierte Region) wurde durch Verdauung mit den Enzymen HindIII für P-FMV, HindIII plus BamHI für ctp2-CP4-EPSPS und EcoRI und PstI für die E9-3'-untranslatierte Region bestimmt. Zusätzliche Experimente wurden mit SacI und XhoI für die P-FMV-ctp2-CP4-EPSPS-Region.und für die E9-3'-Region durchgeführt. Plasmid-DNA, gemischt mit nicht-transgener Zuckerrüben-DNA, und nicht-transgene Zuckerrüben-DNA allein wurden als Positiv- bzw. Negativköntrollen mit denselben Enzymen verdaut.

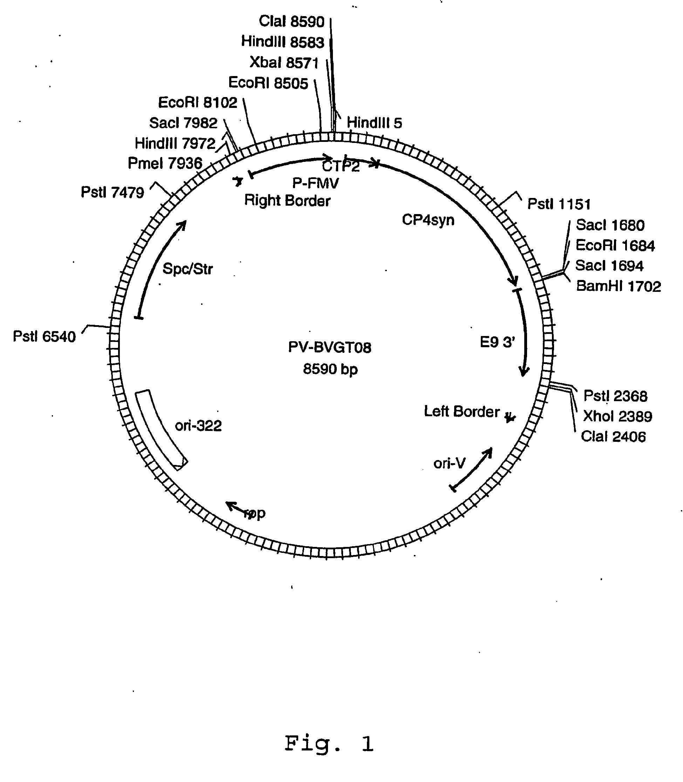

Diese Enzyme schneiden innerhalb des vorgesehenen DNA-Inserts zwischen den linken und rechten T-DNA-Grenzen (siehe die Plasmid-Karte in Fig. 1), so daß, wenn die entsprechenden Elemente intakt sind, die Größe der hybridisierenden Fragmente in H7-1-DNA und PV-BVGT08-DNA identisch sein sollte.

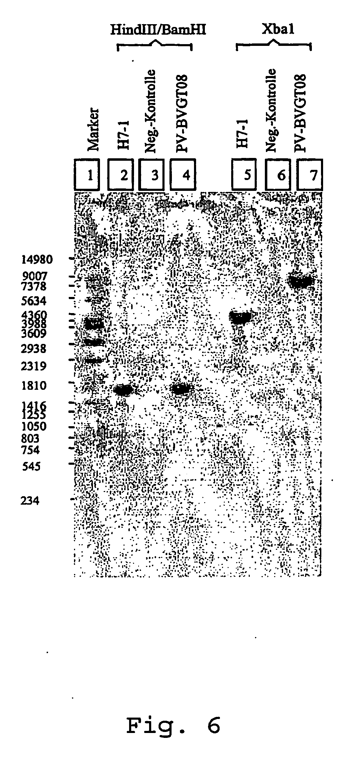

Als zusätzliche Kontrolle wurden die DNAs mit XbaI verdaut. XbaI schneidet einmal zwischen dem Promotor und der kodierenden Region der ctp2-CP4-EPSPS. Man würde daher ein 8,6-kb-Fragment mit der PV-BVGT08-DNA und im Falle von H7-1 ein Grenzfragment, das sich in der Größe im Vergleich zu dem PV-BVGT08-Fragment unterscheidet, erwarten. Die Ergebnisse sind in den Fig. 6, 7 und 8 dargestellt.

Fig. 6: Die Verdauung mit HindIII und BamHI setzte das CP4-EPSPS-Gen frei und der Blot wurde mit einem durch PCR erzeugten CP4-EPSPS-Fragment sondiert. Die Negativkontrolle (Spur 6) zeigte keinerlei Hybridisierungsbanden. Genomische DNA vom Event H7-1 und Plasmid PV-BVGT08, gemischt mit nicht-transgener DNA, bildeten jeweils ein etwa 1,7-kb-Fragment, das der erwarteten Größe entspricht. Die Verdauung mit XbaI führte zu dem erwarteten 8,6-kb-Fragment des linearisierten PV-BVGT08. Für Event H7-1 führte die Verdauung zu einem ungefähr 4,0-kb-Grenzfragment (siehe auch Fig. 4 und 5). Erneut zeigte die Negativkontrolle keinerlei Signal.

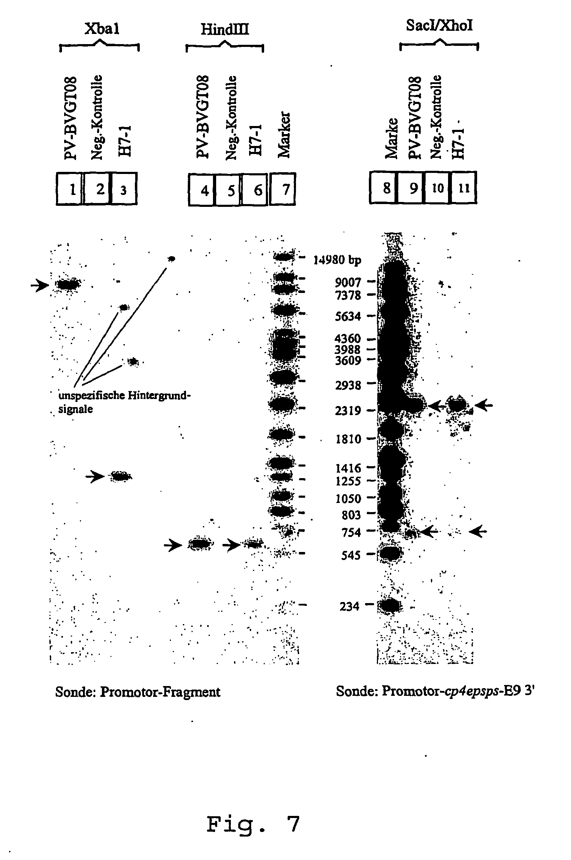

Fig. 7: Die Verdauung mit HindIII setzte den Braunwurz-Mosaik-Virus-Promotor frei und der Blot wurde mit einem durch PCR erzeugten Promotor-Fragment sondiert. Die Negativkontrolle (Spur 5) zeigte keinerlei Hybridisierungssignale. Genomische DNA aus dem Event H7-1 und Plasmid PV-BVGT08, gemischt mit nicht-transgener DNA, bildeten jeweils ein hybridisierendes Fragment mit einer ungefähren Größe von 0,6 kb. Dieses Fragmente entspricht der erwarteten Größe des Promotors (Spur 4 und 6).

Die Verdauung mit XbaI führte zu dem erwarteten 8,6-kb-Fragment des linearisierten PV-BVGT08 und zu dem etwa 1,3 kb großen linken Grenzfragment (Spur 1 und 3) von Event H7-1. Dieses 1,3-kb-Fragment ist auch ein zusätzlicher Beweis dafür, daß das Event H7-1 nur eine einzelne Kopie des Transgens enthält. Erneut zeigte die Negativkontrolle (Spur 2) keinerlei Signal.

Die Verdauung mit SacI/XhoI setzte den Promotor zusammen mit der kodierenden Region der CP4-EPSPS und der Poly-A-Region frei. Hybridisierung mit der vollständigen Promotor-ctp2-CP4-EPSPS-Poly-A-Signal-Kassette (PmeI/XhoI-Fragment) ergab die erwarteten 2,3-kb-Promotor-ctp2-CP4-EPSPS- und 0,7-kb-Poly-A-Signal-Fragmente, sowohl mit der PV-BVGT08-DNA, gemischt mit nicht-transgener DNA, als auch mit genomischer DNA von H7-1 (Spur 9 und 11).

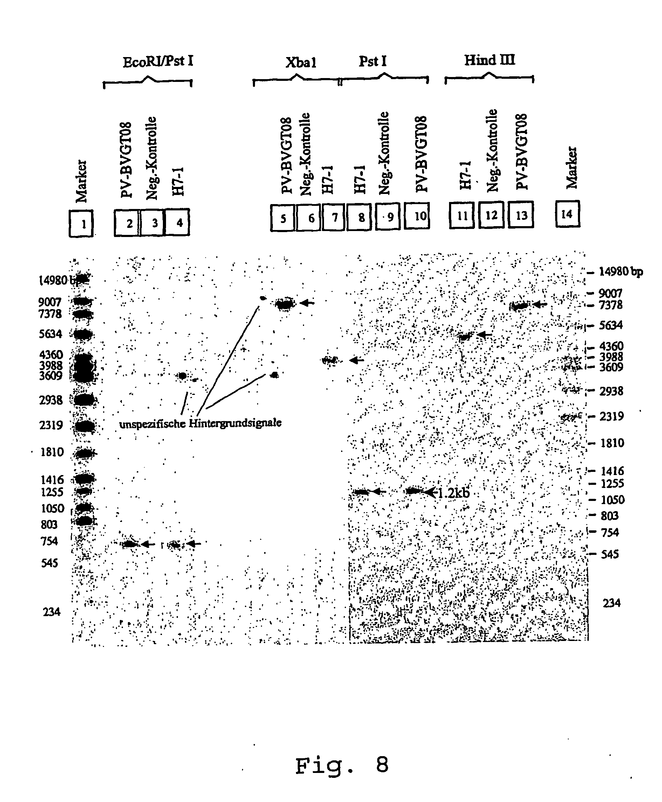

Fig. 8: die Verdauung mit PstI und EcoRI setzte das E9-3'-Polyadenylierungssignal frei und der Blot wurde mit einem Polyadenylierungssignal-Fragment sondiert. Die Negativkontrolle (Spur 3) zeigte keinerlei Hybridisierungsbanden. Plasmid PV-BVGT08, gemischt mit nicht-transgener DNA, und genomische DNA von Event H7-1 bildeten beide ein ungefähr 0,6 kb großes Fragment, das der erwarteten Größe entspricht. Die Verdauung mit XbaI führte zu dem erwarteten 8,6-kb-Fragment des linearisierten PV-BVGT08 und zu einem ungefähr 4,0 kb großen Grenzfragment beim Event H7-1.

Die Verdauung mit PstI setzte das E9-3'-Polyadenylierungssignal, verbunden mit einem 0,5 kb großen 3'-Abschnitt der kodierenden Region der CP4-EPSPS, frei. Das erhaltene 1,2-kb-Fragment war wie erwartet sowohl mit der genomischen H7-1-DNA als auch mit der PV-BVGT08-DNA nachweisbar.

Die Verdauung mit HindIII führte zu einem 8,0-kb-Fragment des linearisierten PV-BVGT08 minus dem Promotorfragment (Spur 13) und zu einem 5,2-kb-Grenzfragment (Spur 11) bei H7-1. Das einzelne 5,2-kb-Fragment aus der HindIII-Verdauung und das einzelne 4,0-kb-Fragment aus der XbaI-Verdauung stellen ebenfalls einen zusätzliche Beleg dafür dar, daß das Event H7-1 nur eine Kopie eingefügter DNA enthält. Erneut zeigte die Negativkontrolle kein Signal.

Zusammengefaßt belegen die Ergebnisse für die Blots daß alle Elemente der transferierten DNA intakt sind und daß das Event H7-1 eine einzelne intakte kodierende Region der CTP2-CP4-EPSPS mit deren regulatorischen Elementen, dem pFMV-Promotor und der E9-3'-Transkriptionsterminator-Sequenz, enthält.

### 4. Analyse zur Bestimmung von "Backbone"-Fragmenten

Die "Backbone"-Region eines Ti-Plasmids wird definiert als die Region außerhalb der T-DNA, begrenzt durch linke und rechte Grenzsequenzen, die aus den ori-Genen und den Selektionsgenen für die Bakterienreplikation und Bakterienselektion bestehen und die durch Agrobakterium-vermittelte Transformation normalerweise nicht in das Pflanzengenom überführt werden. Um die Abwesenheit der "Backbone"-Vektor-DNA in Event H7-1 zu bestätigen, wurde genomische DNA aus H7-1, aus einer nicht transformierten Kontrolle und genomische DNA aus H7-1, gemischt mit PV-BVGT08-DNA, mit dem Restriktionsenzym XbaI verdaut und mit drei überlappenden durch PCR.erzeugte Sonden, die die vollständige "Backbone"-Sequenz umfaßten, untersucht. Eine vierte Sonde weist das vollständige "Backbone" in einem. Fragment auf.

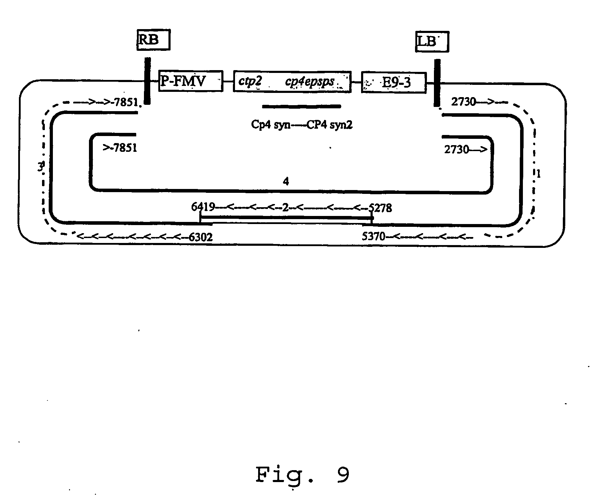

Die verwendeten Sonden repräsentieren die "Backbone"-Sequenz (siehe Fig. 9):
1 : bp 2730- 5370
2 : bp 5278- 6419
3 : bp 6302- 7851
4 : bp 2730- 7851

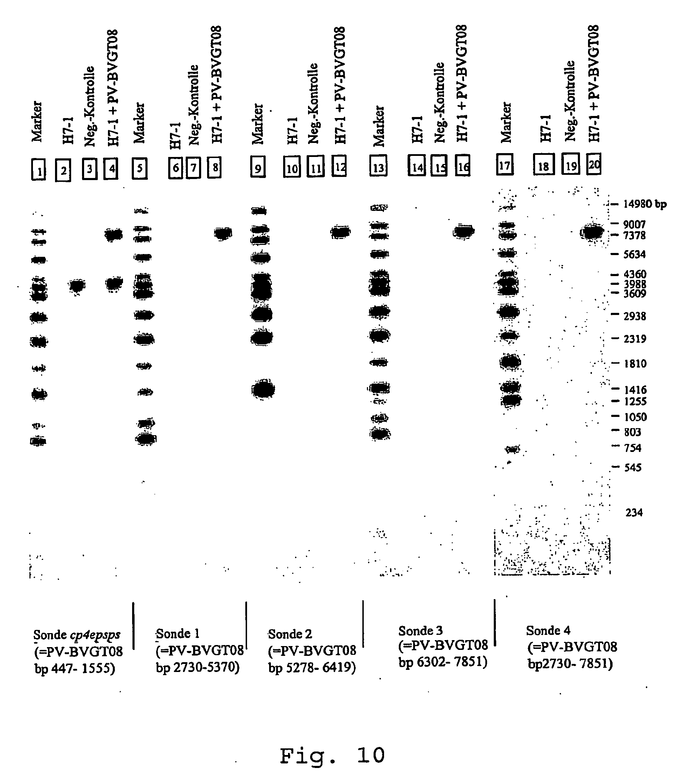

Fig. 10 zeigt das Ergebnis der Southern-Blot-Analyse. Spuren 6, 10, 14 und 18: verdaute genomische DNA von H7-1, die mit den "Backbone"-Fragmenten des vollständigen "Backbones" sondiert wurde, zeigte keinerlei Hybridisierungsbanden. Nur Spuren 4, 8, 12, 16 und 20: genomische DNA von H7-1, gemischt mit PV-BVGT08-DNA, zeigt Banden von 8,6 kb, wie erwartet. Die Banden repräsentieren die linearisierte PV-BVGT08-DNA.

Spuren 2 und 4: genomische DNA von H7-1 und genomische DNA von H7-1, gemischt mit PV-BVGT08, und hybridisiert mit dem CP4-EPSPS-Fragment, zeigte Hybridisierungssignale. Die 4-kb-Bande in Spur 2 repräsentiert das rechte Grenzfragment, die zwei Banden der Spur 4 repräsentieren erneut das 4,0 kb lange rechte Grenzfragment und das 8,6 kb große linearisierte PV-BVGT08-Plasmid. Beide Banden weisen dieselbe Intensität auf. Dies ist ein klarer Hinweis, daß die Konzentration der zugegebenen PV-BVGT08-DNA mit der Konzentration des CP4-EPSPS-Elements in der H7-1-DNA vergleichbar ist. Die Konzentration der verwendeten Plasmid-DNA entspricht 0,5 Kopien. Wenn "Backbone"-Sequenzen im H7-1-Genom integriert wären, sollten klare Signale nachweisbar sein.

Diese Ergebnisse belegen, daß H7-1 keine nachweisbare "Backbone"-Sequenz des für die Transformation verwendeten Plasmids enthält. Dieses Ergebnis wurde auch durch die Daten der Analyse der flankierenden 5'- und 3'-Genombereiche (siehe unten) unterstützt.

### 5. Identifizierung der flankierenden 5'- und 3'-Genomsequenzen

Agrobakterium-vermittelte Transformation führt normalerweise zur Integration aller Sequenzen zwischen der linken und rechten Grenze in das Pflanzengenom. Die 5'- and 3'-Enden der integrierten Plasmid-DNA sollten sich innerhalb oder in der Nähe der linken beziehungsweise rechten Grenzsequenzen befinden. Daher wurde eine Invers-PCR-Technik verwendet, um diese Bereiche zu identifizieren. Die klonierten PCR-Produkte wurden sequenziert und die Sequenzdaten wurden mit der PV-BVGT08-Sequenz verglichen.

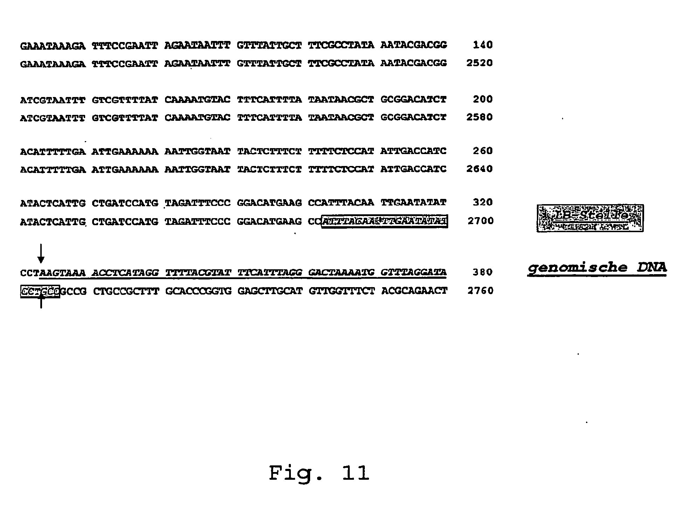

Fig. 11 zeigt den Abgleich der Sequenz des klonierten Invers-PCR-Fragments (D1U.RPT)(= Genom H7-1, obere Sequenz), erhalten mit Primern für die Analyse der linken Grenzregion, mit der PV-BVGT08-Sequenz (unter Sequenz). Der Vergleich der beiden Sequenzen zeigt, daß die Homologie exakt innerhalb der Grenzsequenz endet.

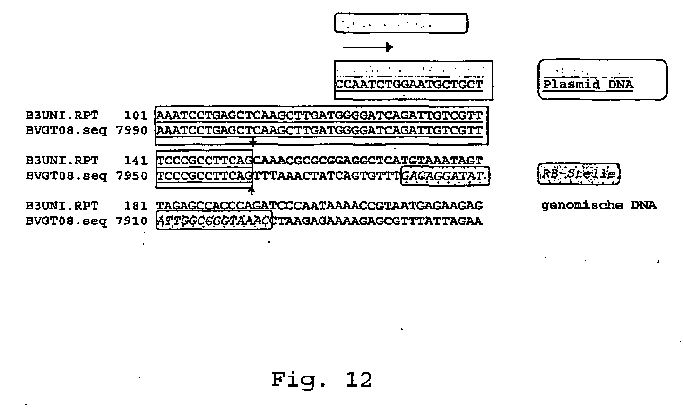

Fig. 12 zeigt den Abgleich der Sequenz des klonierten Invers-PCR-Fragments (B3UNI.RPT)(= Genom H7-1, obere Sequenz), erhalten mit Primern zur Analyse der rechten Grenzregion, gegen die PV-BVGT08-Sequenz (untere Sequenz). Der Vergleich beider Sequenzen zeigt, daß die Homologie bereits 18 Nukleotide vor der Grenzsequenz endet.

Insgesamt ist dies ein klarer Hinweis darauf, daß die Sequenz zwischen den linken und rechten Grenzen des Ti-Plasmids PV-BVGT09 korrekt integriert ist. Die Sequenz endet innerhalb oder unmittelbar vor den Grenzen. Diese Daten unterstützen das Ergebnis der "Backbone"-Analyse, daß keine Sequenzen des "Backbones" außerhalb der Grenzregionen in das H7-1-Genom integriert wurden.

Um festzustellen, ob die flankierenden Sequenzen auf der rechten und linken Seite des Inserts in dem Zuckerrüben-Event H7-1 intakte genomische Sequenzen der Pflanze sind, wurde eine Invers-PCR-Analyse mit den Primer-Kombinationen P1, P2, P3 und P4 durchgeführt.

Die Primer der Primer-Kombinationen P1 und P2 sind außerhalb des Inserts lokalisiert. Wenn die DNA des Insertionsortes innerhalb des Events H7-1 mit der DNA einer nichttransformierten Kontrolle identisch ist, sollte die PCR zu zwei PCR-Fragmenten führen, die die Synthese aus den zwei Primer-Kombinationen repräsentieren. Die Primer der Primer-Kombinationen P3 und P4 sind in einer Weise ausgelegt, daß einer der entsprechenden Primer innerhalb des CP4-EPSPS-Inserts lokalisiert ist und der andere Primer außerhalb des Inserts, innerhalb der genomischen DNA der Pflanze. Daher sollte die PCR ausschließlich Fragmente von der DNA des Events H7-1 bilden.

Sequenzdaten aus der Invers-PCR-Technik, kombiniert mit Daten von dem PV-BVGT08-Vektor, führen zu einer Sequenz, die das H7-1-Insert (PV-BVGT08-Sequenz), die rechten und linken Verbindungsstellenbereiche und zusätzliche genomische Zuckerrüben-DNA enthalten (SEQ ID NO: 5).

Zur Identifizierung der Transgen-zu-Pflanzengenom-DNA-Verbindungsstellen (Identifizierung der Event-Spezifität) und für die Genom-DNA-Regionen auf der linken und rechten Seite des Inserts wurden folgende Primer-Kombinationen verwendet:

P1-Kombination (Primer zur Analyse genomischer DNA außerhalb der rechten Grenzregion, SEQ ID NO: 18, SEQ ID NO: 19):
Oberer Primer: 5'CGG TAA ATG CAT TGG CCT TTG TT
Unterer Primer: 5'CAC CCA GAT CCC AAT AAA ACC GTA AT
Erwartetes PCR-Produkt 241 bp

P2-Kombination (Primer zur Analyse genomischer DNA außerhalb der linken Grenzregion, SEQ ID NO: 20, SEQ ID NO: 21):
Oberer Primer: 5'AAA TGG TTG TAG ATA AAT AAG GAA ATC A
Unterer primer: 5'ACA TGT TTG AGC ACT CTT CTT GT
Erwartetes PCR-Produkt 377 bp

P3-Kombination (Primer zur Analyse der Transgen-Planzengenom-DNA-Verbindungsstelle, SEQ ID NO: 7, SEQ ID NO: 8):
Oberer Primer: 5'ATG CAT TGG CCT TTG TTT TTG AT
Unterer Primer: 5'TGT CGT TTC CCG CCT TCA G
Erwartetes PCR-Produkt 288 bp (SEQ ID NO: 11)

P4-Kombination (Primer zur Analyse der Transgen-Planzengenom-DNA-Verbindungsstelle, SEQ ID NO: 9, SEQ ID NO: 10):
Oberer Primer: 5'CGC TGC GGA CAT CTA CAT TTT TGA AT
Unterer primer: 5'AGT TAA CTT TCC ACT TAT CGG GGC ACT G
Erwartetes PCR-Produkt 751 bp (SEQ ID NO: 12)

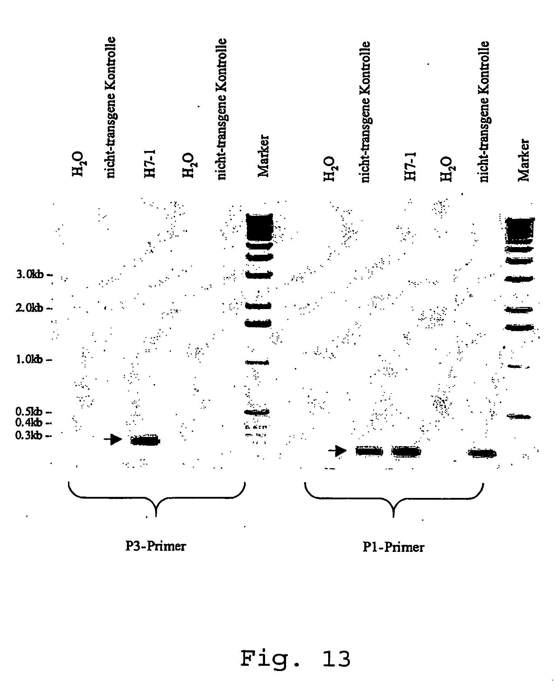

PCR-Experimente mit DNA von Event H7-1 und mit DNA von einer nicht-transgenen Kontrollpflanze unter Verwendung der Primer-Kombination P3, die einen Primer aufweist, der innerhalb des H7-1-Inserts lokalisiert ist, ergab ein Fragment ausschließlich mit der DNA des Events H7-1. Im Gegensatz dazu ergeben PCR-Experimente unter Verwendung der zu Sequenzen außerhalb des Inserts homologen Primer-Kombinatibnen P1 Fragmente sowohl mit Event H7-1 als auch mit nicht-transgener Kontroll-DNA. Siehe Fig. 13 zu diesen Ergebnissen.

Die Ergebnisse zeigen, daß die Sequenz neben der rechten Verbindungsstelle des Inserts in der DNA des transgenen Events H7-1 und in der DNA von nicht-transgenen Pflanzen vorhanden ist. Daraus ergibt sich, daß diese DNA außerhalb des Event-H7-1-Inserts nicht-transgene genomische DNA ist.

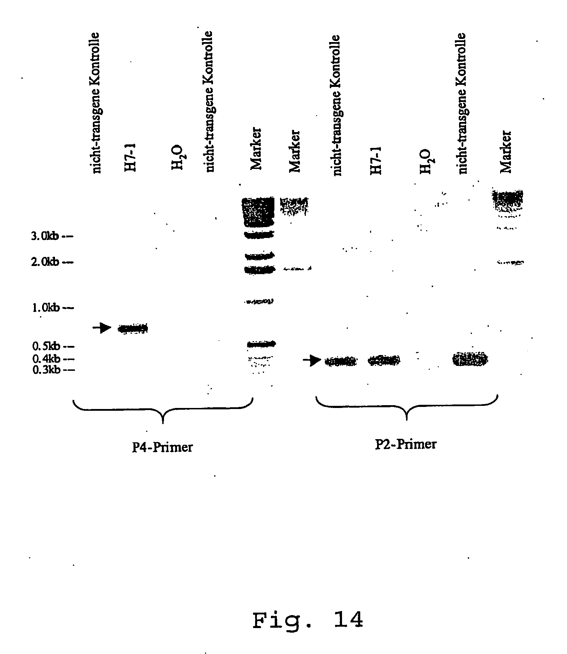

PCR-Experimente, durchgeführt mit Event-H7-1-DNA und DNA aus einer nicht-transgenen Kontrollpflanze unter Verwendung der Primer-Kombination P4, die einen innerhalb des CP4-EPSPS-Inserts lokalisierten Primer aufweist, ergibt ein Fragment ausschließlich mit DNA des Events H7-1. Im Gegensatz dazu ergeben PCR-Experimente unter Verwendung der zu Sequenzen außerhalb des Inserts homologen Primer-Kombination P2 Fragmente sowohl mit DNA des Events H7-1 als auch mit nicht-transgener Kontroll-DNA (Fig. 14).

Die Ergebnisse zeigen, daß die Sequenz neben der linken Verbindungsstelle des Inserts in der DNA des transgenen Events H7-1 und in der DNA von nicht-transgenen Pflanzen vorhanden ist. Daraus ergibt sich, daß diese DNA außerhalb des Event-H7-1-Inserts nicht-transgene genomische DNA ist.

Zusammengefaßt kann festgestellt werden, daß die Sequenzen außerhalb des Inserts des Zuckerrüben-Events H7-1 identisch mit Sequenzen sind, die in nicht-transgenen Pflanzen vorhanden sind. Es kann geschlossen werden, daß diese Sequenzen pflanzliche genomische Sequenzen sind, die in der für die Transformation verwendeten Elternlinie und in anderen konventionellen Zuckerrübenlinien vorhanden sind.

### 6. Stabilität über Generationen

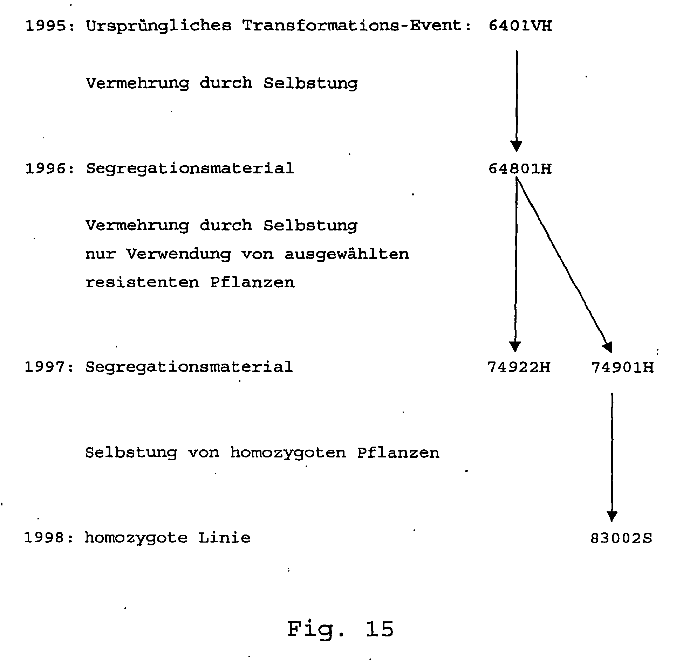

Um die Stabilität der integrierten DNA zu demonstrieren, wurde das ursprüngliche Transformations-Event.H7-1 mit drei Nachkommen (64801H, 74922H und 83002S; siehe Fig. 15) dieser Linie, die durch Selbstbestäubung mit nicht-transgenen Zukkerrübenlinien erhalten wurden, verglichen. Die ursprüngliche transformierte Linie und die Nachkommen wurden in den Jahren 1995, 1996, 1997 und 1998 hergestellt.

Als Kontrolle wurden vier verschiedene nicht-transgene Zukkerrübenlinien (3S0057, 5R7150, 8K1180, 6S0085) analysiert. Alle DNAs wurden mit XbaI, HindIII bzw. BamHI verdaut und mit einem markierten CP4-EPSPS-Fragment hybridisiert. Um zu zeigen, daß die T-DNA stabil in das Pflanzengenom integriert ist, sollten alle Spuren der H7-1-Nachkommen, die mit demselben Restriktionsenzym verdaut wurden, eine Bande von exakt derselben Größe zeigen.

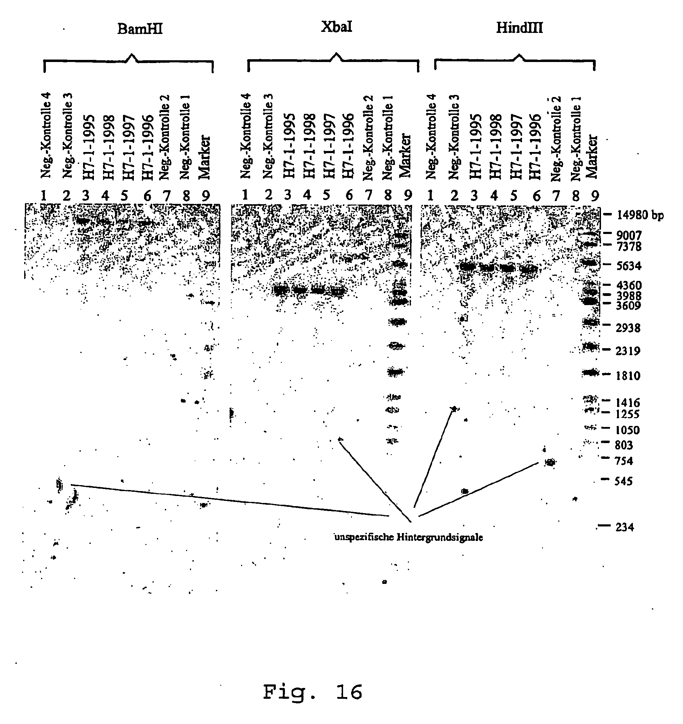

Die DNAs von den H7-1-Nachkommen, Spuren 3 to 6, zeigen die erwarteten Fragmente: DNA verdaut mit BamHI führte zu Banden von ungefähr 11 kb, Verdaus mit XbaI führten zu Fragmenten von 4,0 kb und HindIII-Restriktion bildete Banden von 5,2 kb. Alle Banden von derselben Restriktion, aber aus verschiedenen Jahren, waren in ihrer Größe identisch. Alle nicht-transgenen Linien zeigten keinerlei Signal (Fig. 16).

Diese Ergebnisse zeigen, daß die eingeführte Sequenz stabil in die genomische DNA integriert ist und stabil vererbt wird.

### Sequenzprotokoll - freier Text

SEQ ID: 5
   <223>: Eingefügte DNA mit flankierenden 3'- und 5'-Sequenzen
SEQ ID: 6
   <223>: PCR-Produkt
SEQ ID: 11
   <223>: PCR-Produkt
SEQ ID: 12
   <223>: PCR-Produkt
SEQ ID: 13
   <223>: PCR-Produkt
SEQ ID: 17
   <223>: PCR-Produkt

## Patentansprüche

1. Glyphosat-tolerante Zuckerrübenpflanze, **dadurch gekennzeichnet, daß**
a) ein DNA-Fragment von der genomischen DNA der Zuckerrübenpflanze, Teilen oder Samen davon, durch Polymerasekettenreaktion mit einem ersten Primer, der die Nuklootidsequenz der SEQ ID NO: 3 aufweist, und einem zweiten Primer, der die Nukleotidsequenz der SEQ ID NO: 4 aufweist, amplifiziert werden kann, wobei das DNA-Fragment mindestens 95 % Identität mit der Nukleotidsequenz der SEQ ID NO: 6 aufweist, und/oder
b) ein DNA-Fragnent von der genomischen DNA der Zuckerrübenpflanze, Teilen oder Samen davon, durch Polymerasekettenreaktion mit einem ersten Primer, der die Nukleotidsequenz der SEQ ID NO: 9 aufweist, und einem zweiten Primer, der die Nukleotidsequenz der SEQ ID NO: 10 aufweist, amplifiziert werden kann, wobei das DNA-Fragment mindestens 95 % Identität mit der Nukleotidsequenz der SEQ ID NO: 12 aufweist, und/oder
c) ein DNA-Fragment von der genomischen DNA der Zuckerrübenpflanze, Teilen oder Samen davon, durch Polymerasekettenreaktion mit einem ersten Primer, der die Nukleotidsequenz der SEQ ID NO: 14 aufweist, und einem zweiten Primer, der die Nukleotidsequenz der SEQ ID NO: 16 aufweist, amplifiziert werden kann, wobei das DNA-Fragment mindestens 95 % Identität mit der Nukleotidsequenz der SEQ ID NO: 17 aufweist.

2. Samen einer Pflanze nach Anspruch 1.

3. Zelle, Gewebe oder Teile von der Pflanze nach Anspruch 1.

4. Verfahren zur Identifizierung einer Glyphosat-toleranten Zuckerrübenpflanze, **dadurch gekennzeichnet, daß** das Verfahren den/die Schritt(e) umfaßt des
a) Amplifizierens eines DNA-Fragments von 3500-3900 bp, bevorzugt 3706 bp, von der genomischen DNA der Zuukerzübenpflanze, Teilen oder Samen davon, durch Polymerasekettenreaktion mit einem ersten Primer, der die Nukleotidsequenz der SEQ ID NO: 3 aufweist, und einem zweiten Primer, der die Nukleotidsequenz der SEQ ID NO: 4 aufweist, und/oder
b) Amplifizierens eines DNA-Fragments von 710-790 bp, bevorzugt 751 bp, von der genomischen DNA der Zuckerrübenpflanze, Teilen oder Samen davon, durch Polymerasekettenreaktion mit einem ersten Primer, der die Nukleotidsequenz der SEQ ID NO: 9 aufweist, und einem zweiten Primer, der die Nukleotidsequenz der SEQ ID NO: 10 aufweist, und/oder
c) Amplifizierens eines DNA-Fragments von 990-1100 bp, bevorzugt 1042 bp, von der genomischen DNA der Zuckerrübenpflanze, Teilen oder Samen davon, durch Polymerasekettenreaktion mit einem ersten Primer, der die Nukleotidsequenz der SEQ ID NO: 14 aufweist, und einem zweiten Primer, der die Nukleotidsequenz der SEQ ID NO: 16 aufweist.

5. Test-Kit zur Identifizierung einer transgenen Glyphosat-toleranten Zuckerrübenpflanze, deren Zellen, Gewebe oder Teile, umfassend mindestens ein Primer-Paar mit einem ersten und einem zweiten Primer für eine Polymerasekettenreaktion, wobei der erste Primer eine Sequenz innerhalb der in das Genom der Pflanze aufgenommenen fremden DNA erkennt, und der zweite Primer eine Sequenz innerhalb der flankierenden 3'- oder 5'-Bereiche der DNA erkennt, wobei die Pflanze eine Pflanze nach Anspruch 1 ist.

6. Test-Kit nach Anspruch 5, **dadurch gekennzeichnet, daß**
a) der erste Primer die Nukleotidsequenz der SEQ ID NO: 9 aufweist und der zweite Primer die Nukleotidsequenz der SEQ ID NO: 10 aufweist, und/oder
b) der erste Primer die Nukleotidsequenz der SEO ID NO: 14 aufweist und der zweite Primer die Nukleotidsequenz der SEQ ID NO: 16 aufweist.

## Claims

1. Glyphosate tolerant sugar beet plant, **characterized in that**
a) a DNA fragment from the genomic DNA of the sugar beet plant, parts or seeds thereof, can be amplified by polymerase chain reaction using a first primer having the nucleotide sequence of SEQ ID NO: 3 and a second primer having the nucleotide sequence of SEQ ID NO: 4, whereby the DNA fragment shows at least 95 % identity with the nucleotide sequence of SEQ ID NO: 6, and/or
b) a DNA fragment from the genomic DNA of the sugar beet plant, parts or seeds thereof, can be amplified by polymerase chain reaction using a first primer having the nucleotide sequence of SEQ ID NO: 9 and a second primer having the nucleotide sequence of SEQ ID NO: 10, whereby the DNA fragment shows at least 95 identity with the nucleotide sequence of SEQ ID NO; 12, and/or
c) a DNA fragment from the genomic DNA of the sugar beet plant, parts or seeds thereof, can be amplified by polymerase chain reaction using a first primer having the nucleotide sequence of SEQ ID NO: 14 and a second primer having the nucleotide sequence of SEQ ID NO: 16, whereby the DNA fragment shows at least 95 identity with the nucleotide sequence of SEQ ID NO: 17.

2. Seed of a plant according to claim 1.

3. Cell, tissue or parts from the plant according to claim 1.

4. Method for identification of a glyphosate tolerant sugar beet plant, **characterized in that** the method comprises the step(s) of
a) amplifying a DNA fragment of between 3500-3900 bp, preferably 3706 bp, from the genomic DNA of the sugar beet plant, parts or seeds thereof, using polymerase chain reaction with a first primer having the nucleotide sequence of SEQ ID NO: 3 and a second primer having the nucleotide Sequence of SEQ ID NO: 4, and/or
b) amplifying a DNA fragment of between 710-790 bp, preferably 751 bp, from the genomic DNA of the sugar beet plant, parts or seeds thereof, using polymerase chain reaction with a first primer having the nucleotide sequence of SEQ ID NO: 9 and a second primer having the nucleotide sequence of SEQ ID NO: 10, and/or
c) amplifying a DNA fragment of between 990-1100 bp, preferably 1042 bp, from the genomic DNA of the sugar beet plant, parts or seeds thereof, using polymerase chain reaction with a first primer having the nucleotide sequence of SEQ ID NO: 14 and a second primer having the nucleotide sequence of SEQ ID NO: 16.

5. Test kit for identifying a transgenic glyphosate tolerant sugar beet plant, its cells, tissues or parts, comprising at least one primer par with a first and a second primer for polymerase chain reaction, the first primer recognizing a sequence within the foreign DNA incorporated into the genome of said plant, and the second primer recognizing a sequence within the 3' or 5' flanking regions of the DNA, whereby the plant is a plant according to claim 1.

6. Test kit according to claim 5, **characterized in that**
a) the first primer has the nucleotide sequence of SEQ ID NO: 9 and the second primer has the nucleotide sequence of SEQ ID NO: 10, and/or
b) the first primer has the nucleotide sequence of SEQ ID NO: 14 and the second primer has the nucleotide sequence of SEQ ID NO: 16.

## Revendications

1. Plante de betterave à sucre tolérante au glyphosate, **caractérisé en ce que**
a) un fragment d'ADN de l'ADN génomique de la plante de betterave à sucre, de parties ou de graines de cette dernière peut être amplifié par réaction en chaîne par polymérase avec une première amorce qui présente la séquence nucléotide de la SEQ ID NO: 3 et une deuxième amorce qui présente la séquence nucléotide de la SEQ ID NO: 4, le fragment d'ADN ayant au moins 95 d'identité avec la séquence nucléotide de la SEQ ID NO: 6, et/ou
b) un fragment d'ADN de l'ADN génomique de la plante de betterave à sucre, de parties ou de graines de cette dernière peut être amplifié par réaction en chaîne par polymérase avec une première amorce qui présente la séquence nucléotide de la SEQ ID NO: 9 et une deuxième amorce qui présente la séquence nucléotide de la SEQ ID NO: 10, le fragment d'ADN ayant au moins 95 d'identité avec la séquence nucléotide de la SEQ ID NO: 12, et/ou
c) un fragment d'ADN de l'ADN génomique de la plante de betterave à sucre, de parties ou de graines de cette dernière peut être amplifié par réaction en chaîné par polymérase avec une première amorce qui présente la séquence nucléotide de la SEQ ID NO: 14 et une deuxième amorce qui présente la séquence nucléotide de la SEQ ID NO: 16, le fragment d'ADN ayant au moins 95 % d'identité avec la séquence nucléotide de la SEQ ID NO: 17.

2. Graine de plante selon la revendication 1.

3. Cellule, tissu ou parties de la plante selon la revendication 1.

4. Procédé d'identification d'une plante de betterave à sucre tolérante au glyphosate, **caractérisé en ce que** ce procédé comprend l'étape(les étapes) consistant à :
a) amplifier un fragment d'ADN de 3500 à 3900 bp, de préférence 3706 bp, de l'ADN génomique de la plante de betterave à sucre, de parties ou de graines de cette dernière par réaction en chaîne par polymérase avec une première amorce qui présente la séquence nucléotide de la SEQ ID NO: 3 et une deuxième amorce qui présente la séquence nucléotide de la SEQ ID NO: 4, et/ou
b) amplifier un fragment d'ADN de 710 à 790 bp, de préférence 751 bp, de l'ADN génomique de la plante de betterave à sucre, de parties ou de graines de cette dernière par réaction en chaîne par polymérase avec une première amorce qui présente la séquence nucléotide de la SEQ ID NO: 9 et une deuxième amorce qui présente la séquence nucléotide de la SEQ ID NO: 10, et/ou
c) amplifier un fragment d'ADN 990 à 1100 bp, de préférence 1042 bp, de l'ADN génomique de la plante de betterave à sucre, de parties ou de graines de cette dernière par réaction en chaîne par polymérase avec une première amorce qui présente la séquence nucléotide de la SEQ ID NO: 14 et une deuxième amorce qui présente la séquence nucléotide de la SEQ ID NO: 16.

5. Kit de test pour l'identification d'une plante de betterave à sucre transgénique tolérante au glyphosate, ses cellules, tissus ou parties, contenant au moins une paire d'amorces comprenant une première et une deuxième amorces pour une réaction en chaîne par polymérase, la première amorce détectant une séquence dans laquelle le génome de la plante détecte de l'ADN extérieur absorbé et la deuxième amorce détecte une séquence dans les zones adjacentes 3' ou 5' de l'ADN, la plante étant une plante selon la revendication 1.

6. Kit de test selon la revendication 5, **caractérisé en ce que**
a) la première amorce présente la séquence nucléotide de la SEQ ID NO: 9 et la deuxième amorce la séquence nucléotide de la SEQ ID NO: 10, et/ou
b) la première amorce présente la séquence nucléotide de la SEQ ID NO: 14 et la deuxième amorce la séquence nucléotide de la SEQ ID NO: 16.
